# EUROPÄISCHE PATENTSCHRIFT

(11) **EP 4 410 767 B1**
(45) Veröffentlichungstag und Bekanntmachung des Hinweises auf die Patenterteilung: **01.10.2025**
(21) Anmeldenummer: 23154829.8
(22) Anmeldetag: 03.02.2023
(51) Int. Cl.: C07C 29/151, C07C 67/22, C07C 255/12, C07C 235/06, C07C 69/54, C07C 31/04, C07C 253/00, C07C 231/06, C07C 67/20, C01B 3/02, C01C 1/242, C01C 3/02

(54) **NACHHALTIGES C3 VERFAHREN MIT INTEGRIERTER ERZEUGUNG VON ROHSTOFFEN**
SUSTAINABLE C3 PROCESS WITH INTEGRATED PRODUCTION OF FEEDSTOCK
PROCÉDÉ DURABLE EN C3 AVEC PRODUCTION INTÉGRÉE DE MATIÈRES PREMIÈRES

(43) Veröffentlichungstag der Anmeldung: 07.08.2024
(73) Patentinhaber: Röhm GmbH, 64295 Darmstadt (DE)
(72) Erfinder: KRILL, Steffen, 64367 Mühltal (DE); SCHUMANN, Timm, 64625 Bensheim (DE); PROSCH, Stefan, 60528 Frankfurt (DE); WINGS, Patrick, 50935 Köln (DE); PETER, Patrick, 67574 Osthofen (DE)
(74) Vertreter: Röhm Patent Association

(56) Entgegenhaltungen:
- DE-B- 1 013 636
- US-B2- 9 938 225

## Beschreibung

### Gebiet der Erfindung

Die vorliegende Erfindung betrifft ein neuartiges Verfahren zur Herstellung von organischen Verbindungen mit ein bis drei Kohlenstoffatomen zur Verfügung zu stellen, wobei dieses Verfahren auf nachartige Weise Edukte, die sich aus einer Blausäure-Herstellung, die Teil eines großchemisch nutzbaren Prozesses ist, nutzt. Weiterhin wird die so gewonnene organische Verbindung in dem gleichen Prozess als weiteres Edukt eingesetzt. Insbesondere betrifft das vorliegende Verfahren eine Integration in einen Prozess zur Herstellung von MMA, wobei es sich bei der organischen Verbindung um einen Rohstoff, insbesondere um ein Oxygenat wie Aceton, Methanol, Ethanol oder Propanol handelt.

### Stand der Technik

Weltweit besteht das Ziel, Treibhausgasemission in den kommenden Jahren zu reduzieren und chemische Prozesse als eine relevante Quelle für diese Emissionen zu verbessern. Insbesondere der CO₂-Ausstoß soll vermindert werden. Im Stand der Technik sind bezüglich grundlegenden Aspekten von chemischen Prozessen verschiedene Wege zur Verminderung der Treibhausgasemission beschrieben. Beispielsweise sind Wege beschrieben, ausgestoßenes CO₂ abzufangen und zu organischen Verbindungen umzusetzen, die dann in industriellen Verfahren eingesetzt werden können. Darüber hinaus sind Verfahren beschrieben, die die CO₂-Bilanz in industriellen Verfahren durch Nutzung nichtfossiler Brennstoffe, wie beispielsweise Biogas, verbessern.

Beispielsweise beschreiben Liew et al. (Nature Biotech, Vol. 40, März 2022, S. 335-344) die Herstellung von Aceton und Isopropanol aus Industrieabgasen durch Fermentation. In diesem Verfahren werden CO₂ und Wasserstoff (H₂) aus Industrieanlagen durch das Bakterium *Clostridium autoethanogenum* zu Acteon und Isopropanol umgesetzt.

Es gibt einige großtechnisch in relevanten Mengen hergestellte Chemikalien, die bezüglich dem jeweiligen Treibhausgas-Fußabdruck verbessert werden können. Dabei ist es insbesondere interessant diejenigen Prozesse zu verbessern, die a) mit hohen Kapazitäten eingesetzt werden und b) eine große Komplexität, z.B. bzgl. der Mehrstufigkeit des Verfahrens aufweisen. Zu solchen Molekülen gehören beispielsweise Methylmethacrylat (MMA), Methionin oder Isophoron-Derivate. Ein besonderer Fokus der vorliegenden Erfindung liegt auf der Verbesserung ebendieser Herstellungsprozesse, insbesondere bei der Herstellung von MMA:
Es sind verschiedene Verfahren zur Herstellung von Alkyl(meth)acrylaten, insbesondere von Methylmethacrylat (MMA) bekannt. Üblicherweise gehen die Verfahren von C₂-, C₃- oder C₄-Bausteinen aus.

In US 9,938,225 B2 ist ein Verfahren zur Herstellung von Methylmethacrylat mit reduziertem CO₂-Fußabdruck beschrieben. Dabei wird das zur C₃-basierten Synthese notwendige Aceton durch Fermentation von C₆- und C₅-Zuckern erzeugt.

Ein weltweit verbreitetes, kommerzielles Verfahren basiert auf Aceton als Grundstoff und wird meist als C₃-Verfahren oder ACH-Sulfo-Prozess bezeichnet. Hierbei wird Aceton mit Blausäure (HCN) zum zentralen Zwischenprodukt Acetoncyanhydrin (ACH) umgesetzt. Dieses Zwischenprodukt wird isoliert und für die folgenden Prozessschritte zur Herstellung von Methacrylsäure (MAS) und MMA eingesetzt. Ein solcher Prozess ist beispielsweise in US 4,529,816 beschrieben.

Verfahren zur Herstellung der im C₃-Verfahren umgesetzten Blausäure sind als solche ebenfalls bekannt und beispielsweise das sogenannte BMA-Verfahren, bei dem die Blausäure aus Methan und Ammoniak gewonnen wird, und das Andrussow-Verfahren. Das Andrussow-Verfahren ist beispielsweise in Ullmann's Encyclopedia of Industrial Chemistry, Volume 8, VCH Verlagsgesellschaft, Weinheim 1987, Seiten 161-162 beschrieben. In diesem Verfahren wird das Eduktgasgemisch, das Methan, Ammoniak und Sauerstoff umfasst in einem Reaktor über Katalysatornetze geleitet und bei Temperaturen von etwa 1000 °C umgesetzt. Der notwendige Sauerstoff wird üblicherweise in Form von Luft eingesetzt. Der erhaltene Produktstrom enthält Blausäure (HCN), nicht umgesetzten Ammoniak und Methan, sowie CO, H₂, H₂O und CO₂ als Nebenprodukte.

Das BMA-Verfahren ist beispielsweise in Ullmann's Encyclopedia of Industrial Chemistry, Volume 8, VCH-Verlagsgesellschaft, Weinheim 1987, Seiten 162-163 beschrieben.

Die im Stand der Technik beschriebenen Verfahren weisen relativ hohe Treibhausgasemissionen, insbesondere CO₂-Emissionen, auf. Darüber hinaus erlauben sie keine Wiederverwendung von Abgasen und/oder Nebenprodukten.

### Aufgabe

Der vorliegenden Erfindung lag die Aufgabe zugrunde, ein nachhaltiges Verfahren zur Herstellung von organischen Verbindungen, insbesondere solcher mit ein bis drei Kohlenstoffatomen zur Verfügung zu stellen.

Es bestand dabei insbesondere die Aufgabe, ein solches Verfahren sowohl in Bezug auf die Eduktströme als auch in Bezug auf die Verwendung der organischen Verbindung in einen anderen synthetischen Herstellungsprozess zu integrieren.

Ganz besonders bestand dabei die Aufgabe, das neuartige Verfahren mit einem Herstellungsverfahren für Blausäure zu verknüpfen.

Insbesondere bestand die Aufgabe, als organische Verbindung, Methanol, Ethanol, Propanol oder Aceton, insbesondere Aceton zu realisieren.

Dabei bestand darüber hinaus die Aufgabe, das Verfahren in einen Prozess zur Herstellung eines Alkylmethacrylats, insbesondere MMA zu integrieren.

Weitere nicht explizit genannte Aufgaben können sich im Weiteren direkt oder implizit aus der Beschreibung, den Ansprüchen oder den Beispielen ergeben.

### Lösung

Gelöst wurden die Aufgaben durch die Bereitstellung eines neuartigen Verfahrens zur Herstellung von Blausäure und von organischen Verbindungen, die ein bis drei Kohlenstoffatome und Sauerstoff aufweisen. Dieses Verfahren ist durch das Vorliegen folgender Verfahrensschritte gekennzeichnet:
I. Herstellung einer Mischung von Blausäure und Wasserstoff in einer Reaktionszone A1 eines Reaktors A. Dabei geht man bei dieser Herstellung von Ammoniak und einer Methan-haltigen Fraktion aus. Bei der Methan-haltigen Fraktion kann es sich insbesondere um Erdgas handeln. Die so erhaltene Mischung wird im weiteren als Prozessgas I bezeichnet.
II. Trennung des Prozessgases I in ein überwiegend aus Blausäure bestehendes Prozessgas II und ein überwiegend aus Wasserstoff bestehendes Prozessgas III.
III. Erzeugung eines überwiegend aus CO₂ bestehenden Prozessgases IV in einer Reaktionszone A2 des Reaktors A, ausgehend von einer Methan-haltigen Fraktion.
IV. Mischen mindestens eines Teilstroms des Prozessgases III mit mindestens einem Teilstrom des Prozessgases IV zum Erhalt eines Prozessgases V.
V. Umsetzen des Prozessgases V in einem Reaktor B zur Erzeugung eines Prozessstroms VI, aufweisend die herzustellende organische Verbindung.

Insbesondere vorteilhaft ist das erfindungsgemäße Verfahren einzusetzen, wenn in einem weiteren Verfahrensschritt VI zumindest ein Teils der in Verfahrensschritt I gewonnen Blausäure und mindestens ein Teil der in Verfahrensschritt V gewonnen organischen Verbindung zur Herstellung eines gemeinsamen Folgeprodukts verwendet werden.

Zu den einzelnen Verfahrensschritten gibt es weitere bevorzugte Ausführungsformen, die, wenn nicht anders ausgeführt, auch jeweils miteinander kombiniert werden können. Solche Kombinationen können auch mehrere der folgenden Ausführungsformen in verschiedenen Verfahrensschritten betreffen:

### Zu Verfahrensschritt I:

Verfahrensschritt I wird bevorzugt unter weitgehendem Ausschluss von Sauerstoff in einem Reaktor A in einer Reaktionszone A1 durchgeführt. Besonders effizient ist dies, wenn Reaktionszone A1 dabei einen platinhaltigen Kontakt als heterogenen Katalysator aufweist.

Weiterhin bevorzugt wird das Prozessgas I vor Verfahrensschritt II in einer Absorptionsvorrichtung I mit einem sauren wässrigen Absorptionsmittel derart behandelt, dass verbliebenes Ammoniak abgetrennt wird. Dabei fällt das abgetrennte Ammoniak als wässrige Ammoniumsulfat-haltige Lösung an. Diese Ammoniak-Abtrennung wird im Folgenden auch als Prozessschritt la bezeichnet.

Besonders bevorzugt wird das Ammoniak-abgereicherte Prozessgas II aus Prozessschritt la in mindestens einer Absorptionsvorrichtung II derart mit einem wässrigen, optional carbonylhaltigen Absorptionsmittel behandelt, dass eine wässrige HCN-haltige Lösung erhalten wird. Anschließend kann diese Lösung in mindestens einer Destillationskolonne I derart behandelt werden, dass nach Kondensation eine flüssige, mindestens 99 Gew% Blausäure enthaltende Produktfraktion I erhalten wird.

### Zu Verfahrensschritt II:

Besonders vorteilhaft ist es, wenn bei der Durchführung des erfindungsgemäßen Verfahrens Prozessgas III einen Wasserstoffgehalt größer 90 Vol% aufweist. Besonders bevorzugt wird dieser Gehalt zwischen Verfahrensschritt II und Verfahrensschritt IV optional in einem oder mehreren Aufarbeitungsschritten auf über 95 Vol% erhöht. Vorteilhaft ist es darüber hinaus, wenn dieses optional angereicherte Prozessgas III bei einem Druck unterhalb von 5 bara erhalten wird.

### Zu Verfahrensschritt III:

Bevorzugt handelt es sich bei der Methan-haltigen Fraktionen, die in Verfahrensschritt III in Reaktionszone A2 geleitet wird, um Erdgas. Als besonders vorteilhaft hat es sich dabei erwiesen, dieser Methan-haltigen Fraktionen in oder vor Reaktionszone A2 eine Mischung aus Sauerstoff und Prozessgas Iva, optional mit Wasserstoff zuzuleiten, bevorzugt im Gegenstrom. Besonders bevorzugt wird der Sauerstoff als Gegenstrom zum methanhaltigen Gas direkt in den Reaktor geleitet. Ganz besonders bevorzugt wird reiner Sauerstoff und nicht Luft in den Reaktor eingeleitet. Dies hat den Vorteil, dass die Reaktion in Bezug auf Reaktorzone A2 so gut wie frei von Stickstoff betrieben werden kann und damit die die späteren Prozessgase IV und V frei von störenden Stickoxiden sind.

In diesem Zusammenhang sei auch angemerkt, dass es - wie auch bei Verfahren des Standes der Technik bereits bevorzugt - die methanhaltigen Fraktionen derart auszuwählen, dass diese möglichst schwefelfrei sind, um die Bildung störender Schwefeloxide zu vermeiden. Im Falle von schwefelhaltigen (z.B. Schwefelwasserstoff-haltigen) methanhaltigen Fraktionen ist es zu bevorzugen, diese vor der Einleitung in die Reaktorzonen A1 und A2 zu entschwefeln.

Neben der Erzeugung des Prozessgases IV hat Verfahrensschritt III bevorzugt den Zweck, Wärmeenergie für die endotherme Reaktion des Verfahrensschritts I zur Verfügung zu stellen.

Nach Austritt aus der Reaktionszone A2 weist das Prozessgas IV bevorzugt eine Temperatur zwischen 700 °C und 1300 °C, einen Sauerstoffgehalt von kleiner 5 Vol%, einen NOₓ-Gehalt kleiner 500 Vol-ppm, bevorzugt kleiner 50 Vol-ppm und einen Gehalt an CO, CO₂ und Wasser in Summe von 50 bis 99 Vol%, bevorzugt von 70 bis 98 Vol% und besonders bevorzugt von 80 bis 97 Vol% auf. Danach wird das Prozessgas IV bevorzugt unter Erzeugung von Mitteldruck-Dampf auf eine Temperatur zwischen 150 °C und 450 °C abgekühlt.

Bevorzugt weist Prozessstrom IV einen Sauerstoffgehalt kleiner 0,1 Vol%, besonders bevorzugt kleiner 0,01 Vol% auf.

In einer bevorzugten Ausführungsform der vorliegenden Erfindung kann das Prozessgas IV durch die Umsetzung einer Methan-haltigen Fraktion, insbesondere Erdgas, mit Sauerstoff erhalten werden. Besonders bevorzugt ist es dann, wenn ein Teilstrom dieses Prozessgases IV als ein Prozessgas IVa direkt in Reaktionszone A2 zurückgeführt wird.

Als günstig erwiesen hat es sich dabei, wenn 20 Vol% bis 50 Vol%, besonders bevorzugt 25 Vol% bis 35 Vol% des Prozessgases IV als Prozessgas IVa zurückgeführt werden.

Alternativ dazu und genauso bevorzugt wird ein zweiter Teilstrom IVb des Prozessgases IV in Reaktionszone A2 zurückgeführt. Dabei kann dieses besonders bevorzugt vor oder bei Einleitung in

Reaktorzone A2 erst mit Sauerstoff gemischt werden. Optional, wenn auch weniger bevorzugt kann auch hier - anstelle von Teilstrom IVb - eine Mischung mit der Methan-haltigen Fraktion erfolgen.

Als besonders vorteilhaft hat sich eine Ausführungsform der Erfindung erwiesen, in der ein Teilstrom des Prozessgases IV in Reaktionszone A2 für Verfahrensschritt III und ein zweiter Teilstrom des Prozessgases IV in Reaktor B für Verfahrensschritt V geleitet wird. Dabei hängt das Verhältnis dieser beiden Teilströme zueinander insbesondere von dem jeweiligen Bedarf für die Verfahrensschritte III und V bzw. VI ab, wobei der Verbrauch in Verfahrensschritten V und darauf folgend VI in der Regel maßgebend ist.

Optional können weitere Teilströme des Prozessgases IV abgezweigt werden. Diese können theoretisch, wenn auch nicht bevorzugt, einer Entsorgung oder einer Zwischenlagerung zugeführt werden.

### Zu Verfahrensschritt IV:

In der Regel handelt es sich es sich bei Prozessgas V um ein Synthesegas mit einem Molverhältnis von CO und CO₂ zu Wasserstoff zwischen 1 zu 1 und 1 zu 10, bevorzugt zwischen 1 zu 1,5 und 1 zu 7,5.

Vorteilhaft ist es darüber hinaus, wenn vor Verfahrensschritt IV das Prozessgas IV einer Desoxygenierung unterzogen wird.

Für den Fall, dass Prozessgas IV wie zuvor beschrieben in zwei oder mehrere Teilströme getrennt wird, wird die Desoxygenierung in der Regel nur auf den später in Reaktor B geleitete Teilstrom des Prozessgases IV. Dies betrifft also den Teilstrom, der Verfahrensschritt IV zugeleitet wird.

Alternativ oder additiv, kann auch das Prozessgas V vor Verfahrensschritt V einer Desoxygenierung unterzogen werden.

Besonders bevorzugt erfolgt die Desoxygenierung adiabatisch in Kontakt mit einem Übergangs- oder Edelmetallkatalysator bei einer Temperatur zwischen 200 und 800 °C.

### Zu Verfahrensschritt V:

Bezüglich Verfahrensschritt V und den darauf bevorzugt folgenden Verfahrensschritt VI gibt es diverse Optionen bzw. Ausführungsformen der vorliegenden Erfindung. Die besonders bevorzugten Ausführungsformen der vorliegenden Erfindung werden dabei im Folgenden diskutiert, was aber nicht bedeutet, dass es nicht weitere, auch wirtschaftlich interessante Ausführungen der Verfahrensschritte V und optional VI gibt:
In einer ersten bevorzugten Ausführungsform handelt es sich bei der organischen Verbindung, die in Verfahrensschritt V gebildet wird, um Methanol handelt.

Besonders bevorzugt erfolgt dazu die Synthese des Methanols in Verfahrensschritt V durch Umsetzung in der Gasphase an einem heterogenen Kontakt bei Drücken von 20 bis 120 bar und Temperaturen zwischen 130 °C und 350 °C. Für viele Anwendung wird darauf nach Verfahrensschritt V Prozessstrom VI durch Kondensation in eine Wasserphase, eine überwiegend aus Methanol bestehende Phase und ein nicht kondensiertes Prozessgas VII getrennt.

Die Wasserphase kann teilweise in die Kondensation bzw. in einen darauf folgenden Phasentrenner zurückgeführt werden. Es ist auch möglich, die Wasserphase erst mittels Destillation oder eine Membrantrennstufe aufzukonzentrieren, bevor diese zurückgeführt wird. In der Regel jedoch wird die aufkonzentrierte oder ursprüngliche Wasserphase einer Entsorgung zugeführt.

Prozessgas VII kann einer weiteren Aufarbeitung zugeführt werden, um z.B. noch enthaltenes Methanol zu isolieren. Alternativ kann Prozessgas VII, welches insbesondere C1- bis C3-Bausteine enthält einer der Methan-haltigen Fraktionen des Verfahrensschritts I oder - bevorzugt - III zugeführt werden. Weiterhin ist es auch möglich, Prozessgas VII einer Verbrennung zuzuführen. Auch Kombinationen dieser Vorgehen mittels Teilströmen des Prozessgases VII sind möglich. Bevorzugt jedoch wird das Prozessgas VII mit Prozessgas V gemischt und somit in Reaktor B recycliert.

Die Methanolphase kann optional weiter aufgereinigt werden oder direkt einer weiteren Synthese in Verfahrensschritt VI zugeführt werden.

In dieser ersten Ausführungsform bezüglich Verfahrensschritt V handelt es sich bevorzugt bei Reaktor B um mehrere in Serie durchlaufende Teilreaktoren. Besonders bevorzugt sind dies Rohrbündelreaktoren und/oder Hordenreaktoren. Bei einer solchen Ausgestaltung wird in der Regel in den jeweiligen Teilreaktoren ein Teilumsatz zwischen 20% und 90% erzielt. Als günstig hat es sich dabei erwiesen, wenn die jeweilige Gasmischung vor Eintritt in den jeweiligen nächsten Teilreaktor gekühlt wird.

Bezüglich diesem in der ersten Ausführungsform des Verfahrensschritts V gewonnenen Methanol gibt es für den optionalen, jedoch bevorzugt durchzuführenden Verfahrensschritt VI mehrere Varianten:

In einer ersten besonders bevorzugten Variante der ersten Ausführungsform wird in Verfahrensschritt VI aus der in Verfahrensschritt I hergestellten Blausäure und dem in Verfahrensschritt V gewonnenen Methanol Methylmethacrylat hergestellt. Dabei kann insbesondere das Methanol zu einem Gemisch, enthaltend Schwefelsäure, Ammoniumhydrogensulfat, Methacrylamid und Wasser, bei einer Temperatur zwischen 80 °C und 130 °C zugegeben wurde und das Methacrylamid zuvor unter Einsatz der Blausäure hergestellt wurde. Diese Variante würde somit bedeuten, dass das erfindungsgemäße Verfahren integral in den so genannten und gut beschriebenen ACH-Prozess (siehe z.B. DE 744877 C, EP21 257 06) zur Herstellung von MMA integriert würde.

In einer zweiten Variante wird in Verfahrensschritt VI zunächst aus dem in Verfahrensschritt V gewonnenen Methanol zusammen mit Schwefelwasserstoff Methylmercaptan hergestellt. Der Schwefelwasserstoff kann dazu optional unter Verwendung eines Teilstroms des Prozessgases II hergestellt werden. Das so gewonnene Methylmercaptan kann dann in einem Folgeschritt mit der in Verfahrensschritt I hergestellten Blausäure zu Methionin (siehe z.B. ₂DE 2122491) umgesetzt werden.

In einer zweiten Ausführungsform handelt es sich bei Verfahrensschritt V um eine Fermentation und bei der organischen Verbindung um Aceton. In einer besonders bevorzugten Variante dieser Ausführungsform handelt es sich dann bei dem Folgeprodukt in Verfahrensschritt VI um Methacrylamid, aus welchem wiederum ein Alkylmethacrylat oder Methacrylsäure hergestellt werden kann.

In dieser Variante wird das Aceton besonders bevorzugt bei einer Temperatur zwischen 0 °C und 60 °C mit einem basischen Katalysator mit der Blausäure aus Verfahrensschritt I in Kontakt gebracht. Das so erzeugte Acetoncyanhydrin (ACH) kann dann nach Aufreinigung mit Schwefelsäure, Methanol und Wasser in mindestens zwei Stufen zu einem Methacrylat über die Zwischenstufe des Methacylamids umgesetzt werden.

In einer dritten Ausführungsform handelt es sich bei Verfahrensschritt V um eine Fermentation und bei der organischen Verbindung um Ethanol oder Propanol. In einer bevorzugten Variante dieser dritten Ausführungsform handelt es sich dann bei dem Folgeprodukt in Verfahrensschritt VI um den Ethylmethacrylat oder um Propylmethacrylat handelt, welche durch Veresterung eines auf Blausäure aus Verfahrensschritt I basierenden Methacrylsäureamid oder Methacrylsäure oder welche durch Umesterung eines auf Blausäure aus Verfahrensschritt I basierenden MMA mit dem Ethanol bzw. Propanol aus Verfahrensschritt V erhalten wird.

In einer zweiten Variante der dritten Ausführungsform handelt es sich bei dem Folgeprodukt in Verfahrensschritt VI um Isophorondiamin. Dabei wird das Isophorondiamin durch Umsetzung von Blausäure aus Verfahrensschritt I mit Isophoron, welches zumindest anteilig aus Aceton aus Verfahrensschritt V hergestellt wurde, gewonnen.

Unabhängig von der Ausführungsform bzw. Variante kann Prozessstrom VI vor dem optionalen Verfahrensschritt VI einer weiteren Auftrennung unterzogen werden. Dabei erhält man eine weitere Fraktion, bei der es sich um ein Emissionsgas, das einen Stickoxid Anteil von kleiner 150 Vol-ppm aufweist, handelt.

Zur weiteren Verdeutlichung sei ausgeführt, dass es sich für den Fall, dass es sich bei der organischen Verbindung, die in Verfahrensschritt V gewonnen wird, um Methanol handelt, wird mit diesem Vorgehen nach der Kondensation, aus der wie beschrieben eine wässrige und eine Methanol-haltige Fraktion, sowie ein Prozessgas VII erhalten wird, so eine vierte Fraktion, wie gerade beschrieben, erhalten wird.

### Bezugszeichenliste

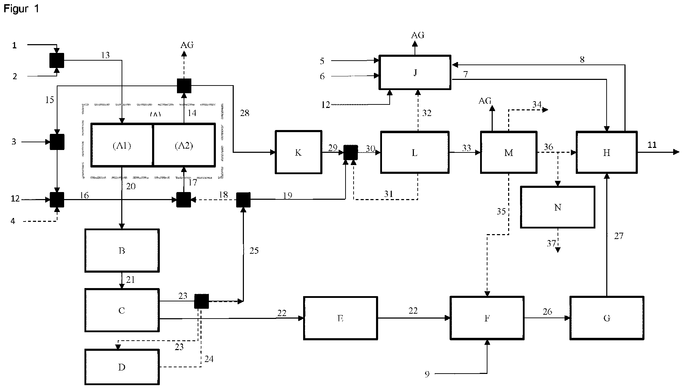

Figur 1 zeigt schematisch die Übersicht des erfindungsgemäßen Verfahren.

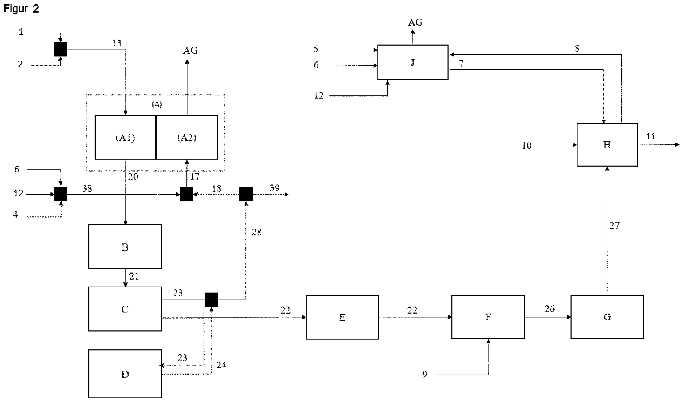

Figur 2 zeigt schematisch das aktuelle Verfahren zeigt, auf das sich das Vergleichsbeispiel bezieht.

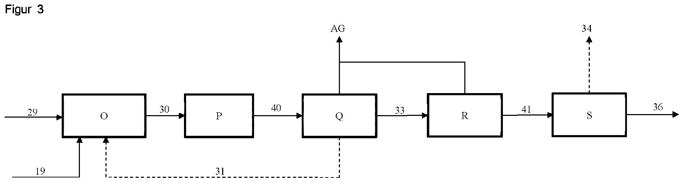

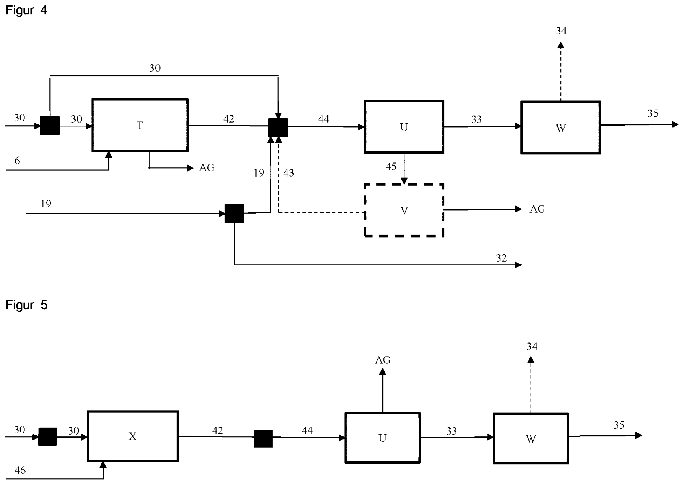

Figuren 3 bis 5 zeigen jeweils eine Detailansicht des Oxygenat-Reaktors und dessen Aufreinigung für die erfindungsgemäßen Beispiel E3 bis E5.

| Fig. 1 | |
|---|---|
| A | BMA-Reaktor |
| A1 | Reaktionsseite BMA-Reaktor |
| A2 | Heizseite BMA-Reaktor |
| B | Absorption I (Schwefelsäurewäsche) |
| C | Absorption II & Destillation I |
| D | Optionale H2-Aufreinigung (Druckwechseladsorption) |
| E | Rein HCN-Tank |
| F | ACH-Reaktor |
| G | ACH-Destillation |
| H | MMA-Reaktor inkl. Aufreinigung |
| J | Schwefelsäure-Reaktor |
| K | Deoxo-Kat |
| L | Reaktor C1-C3 Oxygenat |
| M | Aufreinigung C1-C3 Oxygenat |
| N | Reaktor inkl. Aufreinigung Methylmercaptan |
| 1 | Ammoniak (NH3) |
| 2 | Methan (CH4) |
| 3 | Sauerstoff (O2) |
| 4 | Wasserstoff (H2) |
| 5 | Schwefel |
| 6 | Luft |
| 7 | Schwefelsäure (H2SO4) |
| 8 | Spaltsäure |
| 9 | Frisch-Aceton |
| 11 | MMA |
| 12 | Erdgas |
| 13 | Gasgemisch für Blausäureherstellung |
| 14 | Rauchgas BMA-Reaktor |
| 15 | Rauchgas-Recycle (Prozessgas IV) |
| 16 | Gemisch aus Erdgas, Rauchgasrecyclestrom (Prozessgas IV), Sauerstoff und optional Wasserstoff |
| 17 | Heizgasgemisch mit optionalem Wasserstoff |
| 18 | Optionaler Wasserstoff zur Reaktorbeheizung |
| 19 | Wasserstoff zur Oxygenat-Synthese |
| 20 | Blausäurehaltiges Prozessgas |
| 21 | Prozessgas nach Schwefelsäurewäsche |
| 22 | Reine Blausäure |
| | |
| 23 | Wasserstoffreiches Restgas aus Blausäureproduktion |
| 24 | Optionaler aufgereinigtes wasserstoffreiches Restgas |
| 25 | Wasserstoffreiches Restgas |
| 26 | Roh-ACH |
| 27 | Rein-ACH |
| 28 | CO2-haltiges Abgas aus BMA zur Oxygenat-Synthese |
| 29 | CO2-haltiges Abgas nach Desoxigenierung |
| 30 | Gasgemisch für Oxygenat-Synthese |
| 31 | Optionaler Reaktorrecyclestrom Oxygenat-Synthese |
| 32 | Optionaler Wasserstoff aus Oxygenat-Synthese |
| 33 | Roh-Oxygenat |
| 34 | Optional Ausgeschleustes Oxygenat |
| 35 | Aceton aus Oxygenat-Synthese für ACH-Synthese |
| 36 | Methanol aus Oxygenat-Synthese für MMA-Synthese bzw. Methylmercaptan-Synthese |
| 37 | Folgeprodukte aus Methylmercaptan |
| AG | Abgas |
| | |

| Fig. 2 (zusätzliche Ströme) | |
|---|---|
| 10 | Frisch-Methanol |
| 38 | Gemisch aus Erdgas und Luft und optional Wasserstoff |
| 39 | Optional ausgeschleuster Wasserstoff |
| | |

| Fig 3 (zusätzliche Vorrichtungen und Ströme) | |
|---|---|
| O | Kompressor |
| P | Methanolreaktor |
| Q | Separator |
| R | Vorlaufkolonne |
| S | Produktkolonne |
| 40 | Gemisch aus Synthesegas und Rohmethanol |
| 41 | Entgastes Roh-Methanol |
| | |

| Fig. 4 (zusätzliche Vorrichtungen und Ströme) | |
|---|---|
| T | CO-Generator |
| U | Acetonreaktor |
| V | Druckwechseladsorbtion (PSA) |
| W | Kolonne |
| 42 | CO-reiches Prozessgas |
| 43 | CO-reiches Gas zur Aceton-Synthese aus PSA |
| 44 | Synthesegas in Acetonreaktor |
| 45 | Gasgemisch aus CO und CO2 |
| | |

| Fig. 5 (zusätzliche Vorrichtungen und Ströme) | |
|---|---|
| X | Caphenia-Reaktor |
| 46 | Gemisch aus Erdgas und Wasser |

### Beispiele

Vergleichsbeispiel V1: Herstellung von Blausäure aus Methan und Ammoniak und anschließende Umsetzung zu Methylmethacrylat (MMA) unter Verwendung von nicht prozess-gekoppeltem Methanol

Die Synthese der Blausäure erfolgt nach dem BMA-Verfahren (Details dazu können in DE 1013636 B nachgelesen werden) wie folgt: Die Analyse des Reaktions- und Prozessgases erfolgte mittels online-µGC, welche mit einer Genauigkeit von ± 0.2 % ausgewiesen ist.

Die Darstellung der Blausäure erfolgt im Reaktor A, welcher räumlich in die zwei Teile A1 und A2 getrennt ist.

636 kg/h Synthesemethan, mit einer typischen Zusammensetzung von 95,1 Gew% Methan, 4,47 Gew% Wasserstoff, 0,23 Gew% Kohlenstoffmonoxid, jeweils < 0,1 Gew% Stickstoff, Ethan und Ethylen, werden mit 724 kg/h Ammoniak, mit einer typischen Zusammensetzung von 99,5 Gew% Ammoniak und 0,5 Gew% Wasser, dem Reaktorteil A1 zugeführt und zur Reaktion gebracht.

Der Reaktor A1 besteht aus 1350 mit Platin beschichteten (gemäß DE 3923034, DE 19617040) Reaktionsrohren mit einer Länge von 2100 mm und einem Innendurchmesser von 16 mm, welche in mehreren Kammern mit bis zu je 66 Rohren angeordnet sind. Der Reaktor wird bei einer Temperatur größer 1000 °C und einem Unterdruck von -50 mbarü betrieben. Die Temperatur wird durch Verbrennen von 486 kg/h einer Mischung von handelsüblichem Erdgas, mit einem Methangehalt größer 90 %, und Wasserstoff im Masseverhältnis von Zwei zu Eins mit 9315 kg/h Luft in Reaktorteil A2 erzeugt. Dabei entsteht ein Abgasstrom (Prozessstrom II) von 9818 kg/h, welcher 775 kg/h CO₂ und 2,5 % Restsauerstoff sowie 2000 mg/m³ NOx enthält, welche anschließend durch eine nichtkatalytische Reduktion mit Ammoniak auf 300 mg/m³ reduziert wird. Der Abgasstrom wird anschließend in die Atmosphäre gegeben.

Der den Reaktor A1 verlassende Prozessstrom I hat typischerweise eine Zusammensetzung von 22 Vol% Blausäure, 3 Vol% Ammoniak, 1 Vol% Methan und 74 Vol% Wasserstoff. Dieser wird in einem dem Reaktor zugehörigen Wärmtauscher auf <200°C abgekühlt und bei einem Druck von -25 mbarü der nachfolgenden Absorptionsstufe I zugeführt.

Das Ammoniak wird durch Zuführen in Absorptionsstufe I, ausgeführt als zweistufige Säurewäsche, mittels 30 gew%iger Schwefelsäure aus dem Prozessgasstrom I abgetrennt, wodurch Prozessgasstrom II erhalten wird. 1100 kg/h der dabei entstehende Ammoniumsulfatlösung werden aus dem Prozess ausgeschleust.

Durch Zuführen des Prozessgasstrom II in die Absorptionsstufe II wird die im Prozessgasstrom II enthaltene Blausäure bei einer Temperatur von 8 °C und einem Druck von -25 mbarü, mit 24.000 kg/h

Wasser aus dem Gasstrom ausgewaschen, wodurch 25.000 kg/h 4 Gew%ige wässrige Blausäurelösung und 267 kg/h eines Restgases, mit einer Zusammensetzung von 98 % Wasserstoff, 1 % Methan und 1 % Stickstoff, entstehen. 75 % dieses Stroms werden in den Reaktorteil A2 für die beschriebene Beheizung zurückgeführt.

Die 25.000 kg/h Blausäurelösung werden anschließend mittels Rektifikation in Wasser, welches in die Absorptionsstufe II zurückgeführt wird, und Blausäure, mit einer Reinheit von >99 %, aufgetrennt.

Es entstehen 1000 kg/h Blausäure, was einer Ausbeute, bezogen auf den Methaneinsatz, von 98,4 % entspricht. Zur Herstellung von 1000kg/h HCN, werden 35 GJ/h benötigt.

Die Blausäure wird in einem zweiten Prozessschritt zu Acetoncyanhydrin (ACH) weiterverarbeitet. Dazu werden 1000 kg/h HCN mit 2140 kg/h Aceton und 2 kg/h Diethylamin vermischt und in einem vierstufigen Reaktorsystem bei jeweiligen Temperaturen von 33 °C, 18 °C, 5 °C und -5 °C umgesetzt. Es werden 3650 kg/h Roh-ACH, mit einer Zusammensetzung von 93 % ACH, 2,5% Aceton, 2,5% HCN (Rest inerte, Nebenprodukte), erhalten, welches anschließend in einem zweistufigen Verdampfersystem aufgereinigt wird. In der ersten Stufe werden bei 120 mbar und 82 °C und in der zweiten Stufe bei 40 mbar und 85 °C die Leichtsieder Aceton und HCN als Brüdenstrom abgetrennt und kondensiert. Es wird ein Destillatstrom von 475 kg/h, mit einer Zusammensetzung von 83 % ACH, 15 % Aceton und 2 % HCN, erhalten, welcher in die Reaktionsstufe 1 zurückgeführt wird. Am Sumpfablauf der Verdampfer entsteht ein Rein-ACH-Strom (99,5 %) von 3000 kg/h.

Im dritten Prozessschritt wird ACH zum Methacrylamid umgesetzt. Dazu werden 1834 kg/h ACH mit 4941 kg/h hochkonzentrierter Schwefelsäure bei <100°C in den ersten Amidierungsreaktor gegeben. Für die Produktion der Schwefelsäure werden 550 kg/h Erdgas für die Feuerung benötigt. Zusammen mit der Verbrennung von Restorganik in der Spaltsäure ergeben sich hierbei 2569 kg/h CO₂-Emissionen. Dem Produktstrom werden anschließend weitere 1166 kg/h ACH zugeben und das Gemisch wird einem zweiten Amidierungsreaktor zugeführt. Anschließend wird das Gemisch bei 160 °C und mehreren Minuten Verweilzeit konvertiert. 7933 kg/h des so erhaltenen Amidgemischs, enthaltend 36 Gew% Methacrylamid, werden mit 1125 kg/h Methanol und 1200kg/h Wasser anschließend zur Veresterung einer sechsstufigen Reaktorkaskade zugeführt. Nach der Aufreinigung mittels Roh- und zweistufiger Rein-Rektifikation werden schließlich 3426 kg/h Methylmethacrylat mit einer Ausbeute bezogen auf das eingesetzte ACH von 91 % erhalten.

Bezogen auf die Produktion eines kg MMAs leiten sich Verbrauchszahlen bzw. Einsatzfaktoren für die einzelnen Rohstoffe ab, sowie die Emissionen aus Schwefelsäure Produktion und HCN Synthese (BMA) (siehe Tabelle 1).

Die Nachfolgende Ökobilanzierung soll die Umweltauswirkungen des produzierten MMAs bezgl. der CO₂ Emissionen bewerten mit der Systemgrenze Cradle-to-Gate - sogenannter Product-Carbon-Footprint (PCF). Hierbei werden nicht nur die direkten CO2-Emission aus dem Prozess (Scope 1) sondern auch die CO2-Emissionen aus den Rohstoffvorketten berücksichtigt (Scope 3). .Die indirekten CO₂-Emissionen der Energien (Scope 2) werden vernachlässigt, da die Produktion des elektrischen Stroms aus rein regenerativen Quellen angenommen wird und Dampf als Wärmequelle aus der Exothermie bzw. Abwärme des MMA-Prozesses selbst produziert wird. Alle CO₂-Emissionen werden auf ein 1 kg produziertes MMA bezogen (Funktionelle Einheit FU). Die Datenquelle für die Primärdaten sind aus den Beispielen zu entnehmen, die sekundär Daten, also die spezifischen CO₂-Emissionen der Rohstoffe, stammen von PlasticsEurope (EcoProfiles). Für Methanol wird ein Wert aus der Literatur angesetzt (Methanol-Mangaging greenhouse gas emissions in the production chain by optimizing the resource base; AIMS Energy; DOI: 10.3934/energy.2018.6.1074). Die genannten Rohstoffe sind mehr als 99% der gesamten eingesetzten Masse. Alle anderen Stoffströme werden hier nicht berücksichtigt Generell wurde diese Ökobilanz nach der ISO 14040/14044 durchgeführt. Für die Umweltwirkungsbewertung wurde die Folgeabschätzung des niederländische Forschungszentrum für Umweltwissenschaften Leiden (CML) mit Charakterisierungsfaktoren aus dem Jahr 2011 mit Aktualisierung April 2013 verwendet.

Die Menge an Rohstoffen und ausgestoßenen CO₂-Emissionen beziehen sich auf das oben beschriebene Vergleichsbeispiel für 1000 kg/h Blausäure bzw. 3424 kg/h MMA.

Mit der Menge und den spezifischen Emissionen lassen sich die einzelnen CO₂-Emissionen der Rohstoffe spezifisch wie auch absolut berechnen.

**Tabelle 1: Datengrundlage und Berechnung des Product Carbon Footprints von MMA für das Vergleichsbeispiel V1.**

| | PCF Edukt / kgCO₂äq./kg | Eingesetzte Menge pro Stunde / kg | CO2äq-Emission kgCO₂äq. |
|---|---|---|---|
| **Upstream Emissionen** | | | |
| Methan | 0,52 | 636 | 330 |
| Erdgas (BMA + H2SO4) | 0,52 | 826 | 429 |
| | | 276 +550 | |
| NH₃ | 2,4 | 724 | 1737 |
| Aceton | 1,6 | 2140 | 3424 |
| Methanol (Erdgas basierend) | 0,83 | 1152 | 956 |
| H2 Export | 1,6 | 66,7 | -106,8 |
| **Teilsumme** | | | 6769 |
| | | | |

| **Produktion** | | | |
|---|---|---|---|
| Direkte CO₂-Emissionen (BMA + H2SO4) | | 3344 (775+2569) | 3344 (775+2569) |
| MMA | | 3424 | 10113 |
| PCF | | **2,95 kgCO2äq./kg MMA** | |

Mit dem Quotienten aus total CO2-Emissionen und MMA Produktion ergibt sich ein PCF von 2,95 kgCO2äq./kg MMA.

In Tabelle 1 ist zu sehen, dass ca. 2/3 der CO₂-Emissionen bei der MMA Produktion auf die Rohstoffe zurückzuführen sind. Die direkten Emissionen stammen zu 23% aus dem BMA-Verfahren und zu 76% aus der Schwefelsäureanlage.

### Erfindungsgemäßes Beispiel E1: Herstellung von Blausäure und Methanol aus Methan und Ammoniak sowie deren anschließende Umsetzung zu Methylmethacrylat (MMA) mit reduzierten CO₂ Emissionen.

Im erfindungsgemäßem Beispiel E1 werden analog dem Vergleichsbeispiel V1 636 kg/h Synthesemethan, mit einer typischen Zusammensetzung von 95,1 Gew% Methan, 4,47 Gew% Wasserstoff, 0,23 Gew% Kohlenstoffmonoxid, < 0,1 Gew% Stickstoff, Ethan und Ethylen, mit 724 kg/h Ammoniak, mit einer typischen Zusammensetzung von 99,5 Gew% Ammoniak und 0,5 Gew% Wasser,in den Reaktorteil A1 gegeben. Es entstehen analog dazu 1000 kg/h Blausäure und 267 kg/h des wasserstoffreichen Restgases.

Für die Beheizung des Reaktors wird ein 300 bis 400 °C heißer Rauchgas-Recyclestrom von 3881 kg/h, bestehend aus 55 Gew% CO₂, 44 Gew% H₂O und 1 Gew% O₂, mit 2751 kg/h technisch reinem Sauerstoff sowie 690 kg/h handelsüblichem Erdgas, mit einem Methangehalt > 90 Gew%, im Reaktorteil A2 thermisch umgesetzt. Es entstehen dabei 7323 kg/h eines Rauchgases von dem 53% kontinuierlich abgetrennt werden und für den beschriebenen Rauchgas-Recyclestrom dienen. Der andere Teil des Stroms wird mittels eines Edelmetallkatalysators und eines kohlenstoffhaltigen Reduktionsmittels desoxigeniert. Nach einer Abkühlung auf 20 °C und Abtrennung von auskondensiertem Wasser erhält man einen Strom mit 2019 kg/h mit einem CO₂ Anteil von 98 Gew% und < 25 mg/m³ NOx (Rest Wasser). Mit Rauchgas wird in diesem Zusammenhang des vorliegenden Beispiels der Prozessstrom IV gemäß Beschreibung aufgeführt.

Im erfindungsgemäßen Beispiel E1 wird in einem nächsten Prozessschritt dieser CO₂-Strom mit den 267 kg/h Restgas mit einem H₂-Gehalt von 98 Gew%, welches aus dem Reaktorteil A1 erhalten wurde, vermischt und mittels mehrstufiger Kompression auf 85 bar verdichtet. Zwischen den Kompressionsstufen wird nach der Kühlung kondensiertes Wasser von der Gasphase abgetrennt. Die Zusammensetzung des gemischten Gases beträgt dann 72,1 Vol% H₂, 24,3 Vol% CO₂, und 3,6 Vol% H₂O. Dies entspricht einer Stöchiometriezahl von 1,96. (Stöchiometriezahl der Methanolsynthese definiert wird als: SN = (H₂ - CO₂) / (CO + CO₂) in mol)

Das gemischte und komprimierte Synthesegas wird anschließend zusammen mit dem Kreislaufgas in einen ersten Wärmetauscher geleitet. Die Eintrittstemperatur beträgt hierbei 67 °C und die Austrittstemperatur 220 °C. Das vorgewärmte Gas weist eine Zusammensetzung von 77,2 Vol% H₂, 20,3 Vol% CO₂, 2,1 Vol% CO, 0,28 Vol% MeOH owie 0,11 Vol% H₂O mit einem Volumenstrom von 19061 Nm³/h auf. Das Gas wird anschließend auf die Rohrseite eines Rohrbündel-Reaktors geleitet, bei der unter Wärmeentwicklung an einem Kupfer/Zink/Aluminumoxid-haltigem Katalysator (bspw. Clariant MegaMax MM800) in Pelletform (6 x 4 mm) die Reaktion einsetzt. Die entstehende Wärme wird durch das siedende Wasser bei 40 bar Druck auf der Mantelseite abtransportiert. Die Umsätze im geraden Durchgang für H₂ und CO₂ sind 20 % respektive 25 % bei einer Verweilzeit von 8,7 Sekunden und einer Katalysatorbettporosität von 0,38. Die produzierte Sattdampfmenge beträgt hierbei 500 kg/h. Das reagierte Reaktoraustrittsgas weist eine Austrittstemperatur von 251 °C auf mit einer Zusammensetzung von 69 Vol% H₂, 16,9 Vol% CO₂, 5,8 Vol% MeOH, 5,7 Vol% H₂O und 2,3 Vol% CO sowie einer Volumenstrommenge von 17163 Nm³/h. Das Gas wird weiter in den ersten Wärmetauscher geleitet und auf 130 °C abgekühlt. In dem Schlusskühler wird mittels Kühlwasser (mit einem Vorlauf von 30 °C) das Prozessgas auf 40 °C abgekühlt, wobei flüssige Produkte auskondensieren. Die Flüssigphase (sogenanntes Rohmethanol) weißt bei 83,7 bar eine Zusammensetzung von 62,9 Gew% MeOH, 35,9 Gew% H₂O und 1,12 Gew% gelöstem CO₂ auf. Weitere Nebenprodukte sind 700 Gew-ppm EtOH und 50 Gew-ppm Dimethylether. Der gesamte Massenstrom beträgt 2150 kg/h.

Nach der Abtrennung der Flüssig- und der Gasphase wird das unreagierte Gas mit Hilfe eines Kreislaufkompressors wieder mit dem Synthesegas gemischt. Der Volumenstrom des Kreislaufgases beträgt 15237 Nm³/h Das Kreislaufverhältnis zwischen Kreislaufgas und Synthesegas beträgt hierbei 3,8. Um die Akkumulierung von inerten Komponenten zu verhindern wird aus dem Kreislaufgas ein Spülgasstrom abgezweigt mit einem Massenstrom von 49 kg/h. Der Gesamtumsatz von H₂ beträgt 98%. Das flüssige Rohmethanol wird in einen Rohmethanoltank auf 3 bis 5 bar entspannt, wobei sich gelöste Gase (vor allem CO₂) freisetzen.

In der anschließenden Vorlaufkolonne bei Umgebungsdruck und 75 °C Sumpftemperatur werden weitere Gase (DME) aus dem Rohmethanol abgetrennt. Die Kopftemperatur liegt bei 65,4 °C mit einem Reflux-Verhältnis (mol/mol) von 1.

Alle Abgase vom Spülgasstrom, Rohmethanoltank und Vorlaufkolonne werden zusammen in eine gemeinsame Verbrennungskammer zusammen mit Luft zu CO₂ und H₂O verbrannt. Die entstehenden CO₂ Emission belaufen sich auf 75 kg/h.

Das im Sumpf erhaltene stabilisierte Rohmethanol wird in der nachfolgenden Produktkolonne bei Umgebungsdruck rektifiziert, wobei H₂O im Sumpf mit einer Temperatur von 99 °C, einer Zusammensetzung von 0,11 Gew% MEOH, 376 Gew-ppm EtOH und einem gesamt Massenstrom von 676 kg/h erhalten wird. Das fertige Methanolprodukt im Kopf der Kolonne wird mit einer Temperatur von 64 °C und einem EtOH-Gehalt von 8,2 Gew-ppm gewonnen. Die Produktionsrate liegt bei 1410 kg/h. Das Reflux-Verhältnis der Kolonne beträgt 3,4. Zur Stabilisation der Kolonne und Gewährleistung der Produktsowie Abwasserspezifikation wird durch eine Seitenstromabtrennung im unteren Teil der Kolonne Nebenprodukte (hauptsächlich EtOH) mit einer Zusammensetzung von 92,5 Gew% H₂O, 4,1 Gew% MeOH und 3,3 Gew% EtOH und einem gesamt Massenstrom von 32,9 kg/h abgetrennt.

Das produzierte Methanol wird analog des Vergleichsbeispiels V1, mit Methacrylamid und Wasser zu Methylmethacrylat umgesetzt werden.

**Tabelle 2: Datengrundlage und Berechnung des Product Carbon Footprints von MMA für das Beispiel E1**

| | PCF Edukt / kgCO₂äq./kg | Eingesetzte Menge pro Stunde / kg | CO2äq-Emission / kgCO_{2äq}/h |
|---|---|---|---|
| **Upstream Emissionen** | | | |
| Methan | 0,52 | 636 | 330 |
| Erdgas BMA + H2SO4 | 0,52 | 1240 (690 +550) | 644 |
| NH₃ | 2,4 | 724 | 1737 |
| Aceton | 1,6 | 2140 | 3424 |
| Methanol (Erdgas basierend) | 0,83 | -258 (1152-1410) | -214 |
| O₂ (gasförmig) | 0 | 2751 | 0 |
| **Teilsumme** | | | **5921** |
| H2 export | 1,6 | 0 | 0 |

| **Produktion** | | | |
|---|---|---|---|
| Direkte CO₂-Emissionen (H2SO4 + MeOH) | | 2644 (2569+75) | 2644 |
| MMA | | 3424 | 8565 |
| PCF | | **2,5 kgCO2äq./kg MMA** | |

Mit dem Quotienten aus total CO2-Emissionen und MMA Produktion ergibt sich ein PCH von 2,5 kgCO2äq./kg MMA.

Durch die Konversion von direkten Emissionen der Blausäure mit dem dort erzeugten Wasserstoff lassen sich nicht nur die direkten Emissionen um 21% senken, sondern auch der Scope 3 Anteil wird um 5% reduziert. Die Überproduktion an Methanol, wird hier mit einer CO₂-Gutschrift berechnet. Durch den erhöhten Einsatz an Erdgas wird die Gutschrift von der Methanolproduktion teilweise kompensiert. Die Sauerstoffproduktion über eine cryogene Luftzerlegung erfolgt mit regenerativer elektrischer Energie, so dass hier keine (indirekten) Emissionen anfallen.

Am Ende kann das Beispiel zeigen, dass die CO₂-Emissionen um 15% gegenüber dem Vergleichsbeispiel reduziert werden konnten.

### Erfindungsgemäßes Beispiel E2a: Herstellung von Blausäure und Methanol aus Methan und Ammoniak sowie deren anschließende Umsetzung zu Methylmethacrylat (MMA) unter verbesserten Prozessbedingungen.

Im erfindungsgemäßem Beispiel E2a werden analog dem erfindungsgemäßem Beispiel E1 636 kg/h Synthesemethan, mit einer typischen Zusammensetzung von 95,1 Gew% Methan, 4,47 Gew% Wasserstoff, 0,23 Gew% Kohlenstoffmonoxid, jeweils < 0,1 Gew% Stickstoff, Ethan und Ethylen, mit 724 kg/h Ammoniak, mit einer typischen Zusammensetzung von 99,5 Gew% Ammoniak und 0,5 Gew% Wasser, in den Reaktorteil A1 gegeben. Es entstehen analog dazu 1000 kg/h Blausäure und 267 kg/h des wasserstoffreichen Restgases.

Die Beheizung des Reaktors erfolgt durch unterstöchiometrisches Verbrennen von 690 kg/h handelsüblichem Erdgas, mit einem Methangehalt von über 90 %, mit 2688 kg/h Sauerstoff unter Zugabe von 3809 kg/h eines 300 bis 400 °C heißen Recyclestroms, bestehend aus 55 Gew% CO₂, 44 Gew% H₂O und 1 Gew% CO, in Reaktorteil A2.

Es entstehen dabei 7187 kg/h Rauchgas, von dem 53% abgetrennt werden und für den beschriebenen Rauchgas-Recyclestrom dienen. Nach einer Abkühlung auf 20 °C und Abtrennung von auskondensiertem Wasser werden 1942 kg/h eines Kohlenoxidstrom mit einem CO₂ Anteil von 97 Gew%,einem CO Anteil von 1 Gew%,einem Sauerstoffgehalt < 1000 Gew-ppm und einem NOx-Gehalt < 25 mg/m³ erhalten. Mit Rauchgas wird in diesem Zusammenhang des vorliegenden Beispiels der Prozessstrom IV gemäß Beschreibung aufgeführt.

Analog dem erfindungsgemäßem Beispiel E1 wird anschließend der Kohlenoxidstrom mit dem wasserstoffhaltigen Restgasstrom zu Methanol umgesetzt und dieses dann wie auch die hergestellte Blausäure für die Prodiktion von Methylmethacrylat verwendet.

Die Methanolproduktion steigt dabei um 2% gegenüber dem Beispiel E1 auf 1440 kg/h bei gleichem H₂ Einsatzes.

Die einzelnen Mengenströme sind in der Tabelle 3 für die Analyse des CO₂-Fußabdruckes zusammengefasst

**Tabelle 3: Datengrundlage und Berechnung des Product Carbon Footprints von MMA für das Beispiel E2a**

| | PCF Edukt / kgCO₂äq./kg | Eingesetzte Menge pro Stunde / kg | CO2äq-Emission / kgCO_{2äq}/h |
|---|---|---|---|
| **Upstream Emissionen** | | | |
| Methan | 0,52 | 636 | 330 |
| Erdgas | 0,52 | 1240 (690 +550) | 644 |
| NH₃ | 2,4 | 724 | 1737 |
| Aceton | 1,6 | 2140 | 3424 |
| Methanol (Erdgas basierend) | 0,83 | -288 | -239 |
| | | 1152-1440 | |
| O₂ (gasförmig) | 0 | 2688 | 0 |
| H2 export | 1,6 | 0 | 0 |
| Teilsumme | | | **5896** |
| | | | |

| **Output** | | | |
|---|---|---|---|
| Direkte CO₂-Emissionen (H2SO4 + MeOH) | | 2645 (2569+76) | 2645 (2569+76) |
| MMA | | 3424 | 8541 |
| **PCF** | | **2,49 kgCO2äq/kg MMA** | |

Mit dem Quotienten aus total CO2-Emissionen und MMA Produktion ergibt sich ein leicht niedrigerer PCF von 2,49 kgCO2äq/kg MMA.

### Erfindungsgemäßes Beispiel E2b: Herstellung von Blausäure und Methanol aus Methan und Ammoniak sowie deren anschließende Umsetzung zu Methylmethacrylat (MMA) unter verbesserten Prozessbedingungen.

Im erfindungsgemäßem Beispiel E2b werden analog dem erfindungsgemäßem Beispiel E1 636 kg/h Synthesemethan, mit einer typischen Zusammensetzung von 95,1 Gew% Methan, 4,47 Gew% Wasserstoff, 0,23 Gew% Kohlenstoffmonoxid, jeweils < 0,1 Gew% Stickstoff, Ethan und Ethylen, mit 724 kg/h Ammoniak, mit einer typischen Zusammensetzung von 99,5 Gew% Ammoniak und 0,5 Gew% Wasser, in den Reaktorteil A1 gegeben. Es entstehen analog dazu 1000 kg/h Blausäure und 267 kg/h des wasserstoffreichen Restgases.

Durch weitere Reduktion des C/O-Verhältnisses für die Beheizung des Reaktors unter Beibehaltung der Reaktionswärme bzw. Temperatur konnte überraschenderweise eine parallele Synthesegas Produktion (analog einer partiellen Oxidation) durchgeführt werden.

Hierbei erfolgt die Beheizung des Reaktors analog zu dem Beispiel E2a durch unterstöchiometrisches Verbrennen von 1467 kg/h handelsüblichem Erdgas, mit einem Methangehalt von über 90 %, mit 4032 kg/h Sauerstoff unter Zugabe von 3809 kg/h eines 300 bis 400 °C heißen Recyclestroms, bestehend aus 16 Gew% CO₂, 42 Gew% H₂O, 17 Gew% CO und 24 vol% H2 sowie Reste an Methan, in Reaktorteil A2.

Es entstehen dabei 13746 kg/h Rauchgas bzw. Synthesegase, von dem 40% abgetrennt werden und für den beschriebenen Recyclestrom dienen. Nach einer Abkühlung auf 20 °C und Abtrennung von auskondensiertem Wasser werden 3450 kg/h eines Synthesegasstromes mit einer Zusammensetzung (in vol.%) von 41 H2, 29 CO, 27 CO₂ und 2,3 H₂O sowie Spuren von Methan erhalten. Mit Rauchgas wird in diesem Zusammenhang des vorliegenden Beispiels der Prozessstrom IV gemäß Beschreibung aufgeführt.

Die Mischung zusammen mit wasserstoffhaltigem Restgas ergibt ein Synthesegas mit einer Zusammensetzung von 67 vol%. H2, 16 vol.% CO, 14,9 vol.% CO2 sowie Resten von H₂O und Methan. Analog dem erfindungsgemäßem Beispiel E1 wird anschließend der Synthesegasstrom mit zu Methanol umgesetzt und dieses dann wie auch die hergestellte Blausäure für die Prodiktion von Methylmethacrylat verwendet.

Die Methanolproduktion konnte dabei deutlich auf 2550 kg/hr gegenüber dem Beispiel E1 gesteigert werden. Dies entspricht einer Erhöhung um 77 %. Die Überproduktion an Methanol beträgt 1398 kg/hr.

Die einzelnen Mengenströme sind in der Tabelle 3 für die Analyse des CO₂-Fußabdruckes zusammengefasst

**Tabelle 4: Datengrundlage und Berechnung des Product Carbon Footprints von MMA für das Beispiel E2b**

| | PCF Edukt / kgCO₂äq./kg | Eingesetzte Menge pro Stunde / kg | CO2äq-Emission / kgCO_{2äq}/h |
|---|---|---|---|
| **Upstream Emissionen** | | | |
| Methan | 0,52 | 636 | 330 |
| Erdgas | 0,52 | 2017 (1467 +550) | 1048 |
| NH₃ | 2,4 | 724 | 1737 |
| Aceton | 1,6 | 2140 | 3424 |
| Methanol (Erdgas basierend) | 0,83 | -1398 | -1160 |
| | | 1152-2550 | |
| O₂ (gasförmig) | 0 | 4032 | 0 |
| H2 export | 1,6 | 0 | 0 |
| Teilsumme | | | **5380** |
| | | | |

| **Output** | | | |
|---|---|---|---|
| Direkte CO₂-Emissionen (H2SO4 + MeOH) | | 2645 (2569+76) | 2645 (2569+76) |
| MMA | | 3424 | 8024 |
| **PCF** | | **2,34 kgCO2äq/kg MMA** | |

Das Ergebnis der Analyse der CO2-Emissionen zeigt auf überraschende Weise, dass sich trotz höherer Erdgasmengen, die für die Bereitstellung von Thermischer Energie für die BMA-Reaktion als auch für die Synthesegaserzeugung innerhalb der Brennkammer des BMA-reaktor benötigt wird, die Produktion des Methanols sich positiv für den PCF des MMAs auswirkt. Mit der Annahme, dass die Methanolüberproduktion mit einem konventionellen CO2-Faktor vergütet werden kann, werden hierbei 1160 kg/hr CO2äq. Eingespart. Am Ende liegt der PCF bei diesem Beispiel bei 2,34 kgCO2äq/kg MMA, was einer Reduktion von 22% entspricht.

### Erfindungsgemäßes Beispiel E3: Herstellung von Blausäure und Aceton aus Methan und Ammoniak sowie deren anschließende Umsetzung zu Methylmethacrylat (MMA) mit reduzierten CO₂ Emissionen.

Im erfindungsgemäßem Beispiel E3 werden analog dem erfindungsgemäßem Beispiel E1 636 kg/h Synthesemethan, mit einer typischen Zusammensetzung von 95,1 Gew% Methan, 4,47 Gew% Wasserstoff, 0,23 Gew% Kohlenstoffmonoxid, < 0,1 Gew% Stickstoff, Ethan und Ethylen, mit 724 kg/h Ammoniak, mit einer typischen Zusammensetzung von 99,5 Gew% Ammoniak und 0,5 Gew% Wasser, in den Reaktorteil A1 gegeben. Es entstehen analog dazu 1000 kg/h Blausäure und 267 kg/h des wasserstoffreichen Restgases.

Die Beheizung des Reaktors erfolgt analog dem erfindungsgemäßem Beispiel E1. Nach Abkühlen auf 20 °C und Abtrennung von auskondensiertem Wasser erhält man einen Strom von 2019 kg/h mit einem CO₂ Anteil von 98 Gew% und < 25 mg/m³ NOₓ. Der Rest ist überwiegend Wasser.

62% dieses CO₂-reichen Gasstroms wird zu einem CO-Generator auf Basis einer SOEC (Solid Oxid Electrolysis Cell) (siehe Artikel "Small-Scale CO from CO2 using Electrolysis" in "Chemical Engineering World; Seite 44 - 46; März 2017) zusammen mit elektrischem Strom zu einem CO-reichem Strom konvertiert. Die Zusammensetzung diese Produktstromes beträgt 99 Vol% CO sowie 1 Vol% CO₂ mit einem Volumenstrom von 778 kg/h. Es entstehen weiterhin 438 kg/h Sauerstoff. Der elektrische Stromverbrauch liegt hierbei bei 4,4 MW.

Der CO-reiche Strom wird anschließend zusammen mit einem Teil (5%) des wasserstoffreichem Restgases aus der BMA Anlage, dem Bypass des Kohlendioxidstroms und dem CO Recyclegasstrom zu einem Synthesegas mit einer Zusammensetzung von 50 Vol% CO, 30 Vol% CO₂, 10 Vol% H₂ und 10 Vol% N₂ gemischt. Der Großteil des H₂-Stroms (247 kg/h) wird in die Feuerung der H₂SO₄ Anlage eingesetzt, um das Erdgas dort zu substituieren und somit keine (direkten) zu erzeugen.

Das gemischte Synthesegas wird anschließend in einem Bioreaktor gemäß dem in der Literatur beschriebenen Verfahren (US 2012/0252083, F. E. Liew et al. Carbon-negative production of acetone and isopropanol by gas fermentation at industrial pilot scale, Nature Biotechnology 40, 335-344 (2022)) zu Aceton umgesetzt. Hierbei wird das oben beschriebene Synthesegas mit 1568 Nm³/h bei 35 °C in einem kontinuierlich betriebenen Rührkessel (V=550 m³) bei 1,4 bar, welcher modifizierte Clostridium autoethanogenum enthält, gegeben.

Der Umsatz im geraden Durchgang bezogen auf das CO ist 80% und die Selektivität von CO zu Aceton liegt bei 80%. Es wird ein Produktstrom von 237 kg/h enthalten, welcher aus 80 Gew% Aceton, 15 Gew% Acetessigsäure, 2,5 Gew% Ethanol und 2,5 Gew% iso-Propanol besteht.

Dieses Gemisch wird anschließend mittels Rektifikation aufgereinigt. Als Kopfprodukt fallen 194 kg/h Aceton mit einer Reinheit von 99,5 % an. Als Sumpfprodukt werden 43 kg/h (35 Gew% Aceton, 50 Gew% Acetessigsäure, 7 Gew% Ethanol und 8 Gew% iso-Propanol) ausgeschleust und in einem weiterem Prozessschritt aufgearbeitet.

Das produzierte Aceton wird zusammen mit 1946 kg/h konventionellem Aceton analog des Vergleichsbeispiels V1 erst zu ACH und anschließend weiter zu Methacrylamid und Wasser sowie zu Methylmethacrylat umgesetzt.

Das unreagierte Gas wird zunächst über einen Kompressor auf 15 bar gebracht, um anschließend in eine Druckwechseladsorptionsanlage geleitet zu werden. Hier wird bei einer Effizienz von 90% das CO zurückgewonnen und als Recyclinggasstrom (246 Nm³/h) mit einer Zusammensetzung von 69 Vol% CO und 30 Vol% CO₂ in den Bioreaktor zurückgefahren. Der Tailgasstorm aus der PSA mit einer Zusammensetzung von 76 Vol% CO₂, 17,8 Vol% N₂ und 2,1 Vol% CO und einem Volumenstrom von 873 Nm³/h wird in der H₂SO₄ Anlage thermisch verwertet.

**Tabelle 5: Datengrundlage und Berechnung des Product Carbon Footprints von MMA für das Beispiel E3**

| | PCF Edukt / kgCO₂äq./kg | Eingesetzte Menge pro Stunde / kg | Produkt-Carbon-Footprint / kgCO₂äq. |
|---|---|---|---|
| **Upstream Emissionen** | | | |
| Methan | 0,52 | 636 | 330 |
| Erdgas | 0,52 | 1240 | 644 |
| NH₃ | 2,4 | 724 | 1737 |
| Aceton | 1,6 | 1946 | 3113 |
| Methanol (Erdgas basierend) | 0,83 | 1152 | 954 |
| O₂ (gasförmig) | 0 | 2250 (2699 - 438) | 0 |
| H2 export | 1,6 | 0 | 0 |
| Teilergebnis | | | 6778 |

| **Output** | | | |
|---|---|---|---|
| Direkte CO₂-Emissionen (H2SO4+Aceton) | | 2353 (1324+1029) | 2353 |
| MMA | | 3424 | 9131 |
| **PCF** | | **2,66 kgCO2.äq/kg MMA** | |

Der PCF verringert sich mit diesem Beispiel auf 2,66 kgCO2.äq/kg MMA, was einer Reduktion in Bezug auf das Vergleichsbeispiel von 10 % entspricht.

### Erfindungsgemäßes Beispiel E4: Herstellung von Blausäure und Aceton aus Methan und Ammoniak sowie deren anschließende Umsetzung zu Methylmethacrylat (MMA) mit reduzierten CO₂ Emissionen.

Im erfindungsgemäßem Beispiel E3 werden analog dem erfindungsgemäßem Beispiel E1 636 kg/h Synthesemethan, mit einer typischen Zusammensetzung von 95,1 Gew% Methan, 4,47 Gew% Wasserstoff, 0,23 Gew% Kohlenstoffmonoxid, < 0,1 Gew% Stickstoff, Ethan und Ethylen, mit 724 kg/h Ammoniak, mit einer typischen Zusammensetzung von 99,5 Gew% Ammoniak und 0,5 Gew% Wasser, in den Reaktorteil A1 gegeben. Es entstehen analog dazu 1000 kg/h Blausäure und 267 kg/h des wasserstoffreichen Restgases.

Die Beheizung des Reaktors erfolgt analog dem erfindungsgemäßem Beispiel E1. Nach Abkühlen auf 20 °C und Abtrennung von auskondensiertem Wasser erhält man einen Strom von 2019 kg/h mit einem CO₂ Anteil von 98 Gew% und < 25 mg/m³ NOₓ. Der Rest ist überwiegend Wasser.

Der CO₂-reiche Gasstrom wird zu einem CO-Generator auf Basis einer SOEC (Solid Oxid Electrolysis Cell) (siehe Artikel "Small-Scale CO from CO2 using Electrolysis" in "Chemical Engineering World; Seite 44 - 46; März 2017) zusammen mit elektrischem Strom zu einem CO-reichem Strom konvertiert. Die Zusammensetzung diese Produktstromes beträgt 99 Vol% CO sowie 1 Vol% CO₂ mit einem Massestrom von 1282 kg/h. Es entstehen weiterhin 737 kg/h Sauerstoff, der wiederrum zur Verbrennung im BMA-Reaktor hinzugefügt werden kann. Der elektrische Stromverbrauch liegt hierbei bei 7,4 MW.

Der CO-reiche Strom wird anschließend zusammen mit dem wasserstoffreichen Restgas aus der BMA Anlage zu einem Synthesegas mit einer Zusammensetzung von 61 Vol% H2, 37 Vol% CO, 0,4 Vol% CO₂ und Rest H₂O und N₂ gemischt. Anschließend wird dieses gemischte Synthesegas in einem Bioreaktor gemäß dem in der Literatur beschriebenen Verfahren (US 2012/0252083, F. E. Liew et al. Carbon-negative production of acetone and isopropanol by gas fermentation at industrial pilot scale, Nature Biotechnology 40, 335-344 (2022)) zu Aceton umgesetzt. Hierbei wird das oben beschriebene Synthesegas mit 1549 kg/hr bei 35 °C in einem bzw. mehreren kontinuierlich betriebenen Rührkesseln mit einem gesamten Volumen von 1400 m³ bei 1,4 bar, welcher modifizierte Clostridium autoethanogenum enthält, gegeben. Der Umsatz bezogen auf das CO bzw. H2 ist 80% und die Selektivität von CO zu Aceton liegt bei 80%. Nach dem Abkühlen des Produktstroms auf 5°C und Abtrennung der nicht umgesetzten gasförmigen Edukte (H2 und CO) wird das Prozesswasser zunächst destillativ abgetrennt. Hierbei fällt ein Produktstrom von 385 kg/h an, welcher aus 80 Gew% Aceton, 15 Gew% Acetessigsäure, 2,5 Gew% Ethanol und 2,5 Gew% iso-Propanol besteht.

Dieses Gemisch wird anschließend mittels weiterer Rektifikation aufgereinigt. Als Kopfprodukt fallen 308 kg/h Aceton mit einer Reinheit von 99,5 % an. Als Sumpfprodukt werden 77 kg/h (35 Gew% Aceton, 50 Gew% Acetessigsäure, 7 Gew% Ethanol und 8 Gew% iso-Propanol) ausgeschleust und in einem weiterem Prozessschritt aufgearbeitet.

Das nicht umgesetzte Synthesegas mit einer Zusammensetzung von 75 vol.% H2, 19 vol.% CO sowie Resten von H₂O und CO2 mit einem Massestrom von 864,3 kg/hr wird in der Schwefelsäure-Anlage thermisch umgesetzt und substituiert dort zu 99% das Erdgas. Durch das höhere H/C Verhältnis des Purgegases im Vergleich zum Erdgas sinken die direkten CO2-Emission der H2SO4-Produktion um 14%.

Das produzierte Aceton wird zusammen mit 1795 kg/h konventionellem Aceton analog des

Vergleichsbeispiels V1 erst zu ACH und anschließend weiter zu Methacrylamid und Wasser sowie zu Methylmethacrylat umgesetzt.

**Tabelle 6: Datengrundlage und Berechnung des Product Carbon Footprints von MMA für das Beispiel E4**

| | PCF Edukt / kgCO₂äq./kg | Eingesetzte Menge pro Stunde / kg | Produkt-Carbon-Footprint / kgCO₂äq. |
|---|---|---|---|
| **Upstream Emissionen** | | | |
| Methan | 0,52 | 636 | 330 |
| Erdgas | 0,52 | 782 | 406 |
| NH₃ | 2,4 | 724 | 1737 |
| Aceton | 1,6 | 1832 (2140 - 308) | 2931 |
| Methanol (Erdgas basierend) | 0,83 | 1152 | 954 |
| O₂ (gasförmig) | 0 | 1951 (2688-737) | 0 |
| H2 export | 1,6 | 0 | 0 |
| Teilergebnis | | | 6358 |

| **Output** | | | |
|---|---|---|---|
| Direkte CO₂-Emissionen | | 2357 | 2357 |
| MMA | | 3424 | 8715 |
| **PCF** | | **2,54 kgCO2äq./kg MMA** | |

Dieses Beispiel zeigt, dass sich durch optimierte Bedingung für die biogene Herstellung von Aceton den CO2-Fußabdruck des gesamten MMA-Prozess weiter gemindert werden kann. Der PCF liegt hierbei bei 2,54 kgCO2äq./kg MMA, was einer Reduktion von 14% entspricht.

### Erfindungsgemäßes Beispiel E5: Herstellung von Blausäure und Aceton aus Methan und Ammoniak sowie deren anschließende Umsetzung zu Methylmethacrylat (MMA) mit reduzierten CO₂ Emissionen.

Im erfindungsgemäßem Beispiel E5 werden analog dem erfindungsgemäßem Beispiel E1 636 kg/h Synthesemethan, mit einer typischen Zusammensetzung von 95,1 Gew% Methan, 4,47 Gew% Wasserstoff, 0,23 Gew% Kohlenstoffmonoxid, < 0,1 Gew% Stickstoff, Ethan und Ethylen, mit 724 kg/h Ammoniak, mit einer typischen Zusammensetzung von 99,5 Gew% Ammoniak und 0,5 Gew% Wasser, in den Reaktorteil A1 gegeben. Es entstehen analog dazu 1000 kg/h Blausäure und 267 kg/h des wasserstoffreichen Restgases.

Die Beheizung des Reaktors erfolgt analog dem erfindungsgemäßem Beispiel E1. Nach Abkühlen auf 20 °C und Abtrennung von auskondensiertem Wasser erhält man einen Strom von 2019 kg/h mit einem CO₂ Anteil von 98 Gew% und < 25 mg/m³ NOₓ. Der Rest ist überwiegend Wasser.

Der CO2-reiche Gasstrom wird anschließend einer partiellen Oxidationseinheit mit integriertem elektrischen Plasmalichtbogen zu geführt (siehe Caphenia Patent US 10,906,806 bzw. US 10,927,007). Zusammen mit 1101 kg/hr Erdgas und 1198 kg/hr Wasserdampf (200°C) wird bei einer Temperatur von 1000°C und 20 bar ein Synthesegas mit 58 vol.% H2, 34 vol.% CO und 4,9 vol.% CO2 (Rest Methan) hergestellt. Das so hergestellte Synthesegas eignet sich mit seinem H2 zu CO Verhältnis von 5 zu 3 optimal zur Herstellung von Aceton. Der Strom Bedarf für die Erzeugung des Plasmas beträgt 7,1 MW.

Das Verfahren der Aceton Herstellung ist Analog zu dem Verfahren in Beispiel E4 beschrieben. Zusätzlich wird in diesem Beispiel das unreagierte Purgegas mit Hilfe eines Gebläses wieder in den Bioreaktor zurückgeführt. Dadurch steigert sich der gesamt CO Umsatz auf 95% bei gelichbleibender Selektivität von CO zu Aceton von 80%. Nach der Produktaufreinigung (analog zum Beispiel E4) erhält man einen Produktstrom von 1276 kg/hr Aceton.

Das entstandene Purge mit 958 kg/hr und einer Zusammensetzung von 30 vol.% H2, 36 vol.% CO₂, 13 vol.% sowie 12 vol.% Methan wird in die Feuerung der H2SO4-Anlage geleitet und ersetzt dort 40% des Erdgases für die Stützfeuerung.

Ein Teil des wasserstoffreiche Stroms (134 kg/hr 50%) aus dem BMA-Reaktor wird ebenfalls für die Feuerung der H2SO4 Anlage verwendet und ersetzt dort die restlichen 60% des Erdgases. Die andere Hälfte 134 kg/hr ist für den Export bestimmt und wird mit einem CO2äq-Faktor von 1,6 kgCO2äq./kg vergütet.

Das produzierte Aceton wird zusammen mit 864 kg/h konventionellem Aceton analog des Vergleichsbeispiels V1 erst zu ACH und anschließend weiter zu Methacrylamid und Wasser sowie zu Methylmethacrylat umgesetzt.

**Tabelle 7: Datengrundlage und Berechnung des Product Carbon Footprints von MMA für das Beispiel E5.**

| | PCF Edukt / kgCO₂äq./kg | Eingesetzte Menge pro Stunde / kg | Produkt-Carbon-Footprint / kgCO₂äq. |
|---|---|---|---|
| **Upstream Emissionen** | | | |
| Methan | 0,52 | 636 | 330 |
| Erdgas | 0,52 | 1791 | 931 |
| NH₃ | 2,4 | 724 | 1737 |
| Aceton | 1,6 | 864 (2140 - 1276) | 872 |
| Methanol (Erdgas basierend) | 0,83 | 1152 | 956 |
| O₂ (gasförmig) | 0 | 2751 | 0 |
| H2 export | 1,6 | 134 | -214 |
| Teilergebnis | | | 5122 |

| **Output** | | | |
|---|---|---|---|
| Direkte CO₂-Emissionen | | 1931 | 1931 |
| MMA | | 3424 | 6919 |
| **PCF** | | **2,06 kgCO2äq./kg MMA** | |

Dieses Beispiel zeigt, dass durch die Co-Reformierung des CO2s mit Erdgas, Wasserdampf und elektrischem Strom der PCF deutlich gesenkt werden. Dieser liegt bei **2,06 kgCO2äq./kg MMA,** was einer Reduktion von 31% in Bezug auf das Vergleichsspiel entspricht.

Zusammenfassung der Ergebnisse: (siehe Tabelle 8)

Die in Tabelle 8 dargestellten Ergebnissen zu den Vergleichs- sowie Patentbeispielen zeigen, dass alle Beispiele die Aufgabe der CO2-Reduzierung für die Scope 1 sowie Scope 3 Emissionen erfüllen konnten, trotz erhöhtem Erdgas Bedarfs. Da die Produktion des Sauerstoffes und die Konvertierung des CO2 zu CO bei den Beispiel E3 bis E5 auf Basis von weites gehend CO2-freier elektrischer Energie erfolgt, kann man die Scope 2 Emissionen der Energien hier vernachlässigen.

Weiterhin ist festzustellen, dass durch die Luftfreie bzw. N2-freie Fahrweise des BMA-Reaktorofens eine deutliche Reduzierung der NOx Emissionen erzielt, was andern falls nur mit einer zusätzlichen Ammoniak-Entstickung realisieren lassen würde.

**Tabelle 8: Ergebnisse gemäß Vergleichsbeispiel V1 und erfindungsgemäßen Beispielen E1-E5.**

| **Ausgewählte Prozessparameter Blausäureproduktion** | Vergleichs beispiel V1 | Erfindungsgemäße Beispiele | | | | | |
|---|---|---|---|---|---|---|---|
| | | E1 | E2a | E2b | E3 | E4 | E5 |
| HCN Produktion in kg/h | 1000 | 1000 | 1000 | 1000 | 1000 | 1000 | 1000 |
| CO₂-Emission (BMA Verfahren) / kg/h | 747 | 0 | 0 | 0 | 0 | 0 | 0 |
| Spez. Energiebedarf GJ/t HCN | 35 | 28 | 28 | 60 | 28 | 28 | 28 |
| NOₓ-Emission / mg/m³ | 300 | 20 | 18 | 18 | 20 | 20 | 20 |
| Erdgasverbrauchin kg/h | 334 | 690 | 690 | 1467 | 690 | 690 | 1791 |
| Luft-Bedarf in kg/h | 9315 | 0 | 0 | 0 | 0 | 0 | 0 |
| Rein-O₂ Bedarf in kg/h | 0 | 2751 | 2688 | 4032 | 2250 | 1951 | 2751 |
| Restsauerstoffkonzentration Rauchgas (Prozessgas IV) in % | 2,5 | 1 | 0 | 0 | 1 | 0 | 1 |

| **Ausgewählte Prozessparameter H₂SO₄-Produktion** | | | | | | | |
|---|---|---|---|---|---|---|---|
| H₂SO₄ Produktion in kg/h | 4941 | 4941 | 4941 | 4941 | 4941 | 4941 | 4941 |
| Erdgasverbrauch in kg/h | 550 | 550 | 550 | 550 | 0 | 18 | 0 |
| in kg/h | 2569 | 2569 | 2569 | 2569 | 1029 | 2357 | 1931 |

| **Ausgewählte Prozessparameter MMA-Produktion** | | | | | | | |
|---|---|---|---|---|---|---|---|
| MMA-Produktion (>99.85%) in kg/hr | 3426 | 3426 | 3426 | 3426 | 3426 | 3426 | 3426 |
| Methanol Bedarf für MMA-Produktion in kg/h | 1125 | 1125 | 1125 | 1125 | 1125 | 1125 | 1125 |
| Aceton Bedarf für MMA-Produktion in kg/h | 2140 | 2140 | 2140 | 2140 | 2140 | 2140 | 2140 |

| **Ausgewählte Prozessparameter Oxygenat-Produktion** | | | | | | | |
|---|---|---|---|---|---|---|---|
| Aceton aus BMA-Abgas in kg/h | 0 | 0 | 0 | 0 | 194 | 308 | 1276 |
| Methanol aus BMA-Abgas in kg/h | 0 | 1410 | 1440 | 2550 | 0 | 0 | 0 |
| CO₂ Emissionen aus Oxygenat-Herstellung / kg/h | 0 | 75 | 75 | 75 | 1324 | 0 | 0 |

| **Product Carbon Footprint** | | | | | | | |
|---|---|---|---|---|---|---|---|
| PCF MMA in kg CO_{2ä}q/kg MMA | 2,98 | 2,5 | 2,49 | 2,35 | 2,66 | 2,54 | 2,06 |

## Patentansprüche

1. Verfahren zur Herstellung von Blausäure und organischen Verbindungen, aufweisend ein bis drei Kohlenstoffatome und Sauerstoff, **dadurch gekennzeichnet, dass** das Verfahren die folgenden Verfahrensschritte umfasst:
I. Herstellung einer Mischung von Blausäure und Wasserstoff als Prozessgas I in einer Reaktionszone A1 eines Reaktors A, ausgehend von Ammoniak und einer Methan-haltigen Fraktion, insbesondere Erdgas,
II. Trennung des Prozessgases I in ein überwiegend aus Blausäure bestehendes Prozessgas II und ein überwiegend aus Wasserstoff bestehendes Prozessgas III,
III. Erzeugung eines überwiegend aus CO₂ bestehenden Prozessgases IV in einer Reaktionszone A2 des Reaktors A, ausgehend von einer Methan-haltigen Fraktion,
IV. Mischen mindestens eines Teilstroms des Prozessgases III mit mindestens einem Teilstrom des Prozessgases IV zum Erhalt eines Prozessgases V, und
V. Umsetzen des Prozessgases V in einem Reaktor B zur Erzeugung eines Prozessstroms VI, aufweisend eine organische Verbindung.

2. Verfahren gemäß Anspruch 1, **dadurch gekennzeichnet, dass** in einem Verfahrensschritt VI zumindest ein Teils der in Verfahrensschritt I gewonnen Blausäure und mindestens ein Teil der in Verfahrensschritt V gewonnen organischen Verbindung zur Herstellung eines gemeinsamen Folgeprodukts verwendet werden.

3. Verfahren gemäß Anspruch 1 oder 2, **dadurch gekennzeichnet, dass** Prozessgas IV durch die Umsetzung einer Methan-haltigen Fraktion, insbesondere Erdgas, mit Sauerstoff erhalten wird, dass ein Teilstrom dieses Prozessgases IV als Prozessgas IVa direkt in Reaktionszone A2 zurückgeführt wird und optional erst mit Sauerstoff und danach optional mit weiterer Methanhaltigen Fraktion gemischt wird, bevor diese Gasmischung als Prozessgas IVb in Reaktionszone A2 zurückgeführt wird.

4. Verfahren gemäß mindestens einem der Ansprüche 1 bis 3, **dadurch gekennzeichnet, dass** Verfahrensschritt I unter weitgehendem Ausschluss von Sauerstoff in einem Reaktor A in einer Reaktionszone A1, aufweisend einen platinhaltigen Kontakt, erfolgt, und dass das Prozessgas I vor Verfahrensschritt II in einer Absorptionsvorrichtung I mit einem sauren wässrigen Absorptionsmittel derart behandelt wird, dass verbliebenes Ammoniak abgetrennt wird und dabei als wässrige Ammoniumsulfat-haltige Lösung anfällt.

5. Verfahren gemäß Anspruch 4, **dadurch gekennzeichnet, dass** das Ammoniak-abgereicherte Prozessgas II in mindestens einer Absorptionsvorrichtung II derart mit einem wässrigen, optional carbonylhaltigen Absorptionsmittel behandelt wird, dass eine wässrige HCN-haltige Lösung erhalten wird, und dass diese Lösung anschließend in mindestens einer Destillationskolonne I derart behandelt wird, dass nach Kondensation eine flüssige, mindestens 99 Gew% Blausäure enthaltende Produktfraktion I erhalten wird.

6. Verfahren gemäß mindestens einem der Ansprüche 1 bis 5, **dadurch gekennzeichnet, dass** es sich bei Prozessgas V um ein Synthesegas mit einem Molverhältnis von CO und CO₂ zu Wasserstoff zwischen 1 zu 1 und 1 zu 10 handelt.

7. Verfahren gemäß mindestens einem der Ansprüche 1 bis 6, **dadurch gekennzeichnet, dass** Prozessgas III einen Wasserstoffgehalt > 90 Vol% aufweist, und dass dieser Gehalt zwischen Verfahrensschritt II und Verfahrensschritt IV optional in einem oder mehreren Aufarbeitungsschritten auf > 95 Vol% erhöht wird, und dass das optional angereicherte Prozessgas III bei einem Druck < 5 bara erhalten wird.

8. Verfahren gemäß mindestens einem der Ansprüche 1 bis 7, **dadurch gekennzeichnet, dass** es sich bei den Methan-haltigen Fraktionen um Erdgas handelt, wobei der Methan-haltigen Fraktionen in oder vor Reaktionszone A2 eine Mischung aus Sauerstoff und Prozessgas IVa, optional mit Wasserstoff zugeleitet wird, und dass Verfahrensschritt III dazu dient, Wärmeenergie für die endotherme Reaktion des Verfahrensschritts I zur Verfügung zu stellen.

9. Verfahren gemäß mindestens einem der Ansprüche 1 bis 8, **dadurch gekennzeichnet, dass** das Prozessgas IV nach Austritt aus der Reaktionszone A2 eine Temperatur zwischen 700 °C und 1300 °C, einen Sauerstoffgehalt von kleiner 5 Vol%, einen NOₓ-Gehalt kleiner 500 Vol-ppm und einen Gehalt an CO, CO₂ und Wasser in Summe von 50 bis 99 Vol%, aufweist, und dass das Prozessgas IV danach unter Erzeugung von Mitteldruck-Dampf auf eine Temperatur zwischen 150 °C und 450 °C abgekühlt wird.

10. Verfahren gemäß mindestens einem der Ansprüche 1 bis 9, **dadurch gekennzeichnet, dass** Prozessstrom VI einen Sauerstoffgehalt kleiner 0,1 Vol%, bevorzugt kleiner 0,01 Vol% aufweist.

11. Verfahren gemäß mindestens einem der Ansprüche 1 bis 10, **dadurch gekennzeichnet, dass** ein Teilstrom des Prozessgases IV in Reaktionszone A2 für Verfahrensschritt III und ein zweiter Teilstrom des Prozessgases IV in Reaktor B für Verfahrensschritt V geleitet wird.

12. Verfahren gemäß mindestens einem der Ansprüche 1 bis 11, **dadurch gekennzeichnet, dass** vor Verfahrensschritt IV das Prozessgas IV oder ein später in Reaktor B geleiteter Teilstrom des Prozessgases IV oder vor Verfahrensschritt V das Prozessgas V einer Desoxygenierung, bevorzugt adiabatisch in Kontakt mit einem Übergangs- oder Edelmetallkatalysator bei einer Temperatur zwischen 200 und 800 °C, unterzogen wird.

13. Verfahren gemäß mindestens einem der Ansprüche 2 bis 12, **dadurch gekennzeichnet, dass** es sich bei der organischen Verbindung, die in Verfahrensschritt V gebildet wird, um Methanol handelt, wobei die Umsetzung in Verfahrensschritt V durch Umsetzung in der Gasphase an einem heterogenen Kontakt bei Drücken von 20 bis 120 bar und einer Temperaturen zwischen 130 °C und 350 °C erfolgt, und wobei nach Verfahrensschritt V Prozessstrom VI durch Kondensation in eine Wasserphase, eine überwiegend aus Methanol bestehende Phase und ein nicht kondensiertes Prozessgas VII getrennt wird.

14. Verfahren gemäß mindestens Anspruch 13, **dadurch gekennzeichnet, dass** das Prozessgas VII mit Prozessgas V gemischt wird und, und dass es sich bei Reaktor B um mehrere in Serie durchlaufende Teilreaktoren, bevorzugt Rohrbündelreaktoren und/oder Hordenreaktoren handelt, wobei in den jeweiligen Teilreaktoren ein Teilumsatz zwischen 20% und 90% erzielt wird, und wobei die jeweilige Gasmischung vor Eintritt in den jeweiligen nächsten Teilreaktor gekühlt wird.

15. Verfahren gemäß Anspruch 13 oder 14, **dadurch gekennzeichnet, dass** in Verfahrensschritt VI aus der in Verfahrensschritt I hergestellten Blausäure und dem in Verfahrensschritt V gewonnenen Methanol Methylmethacrylat hergestellt wird, wobei das Methanol zu einem Gemisch, enthaltend Schwefelsäure, Ammoniumhydrogensulfat, Methacrylamid und Wasser, bei einer Temperatur zwischen 80 °C und 130 °C zugegeben wurde und das Methacrylamid zuvor unter Einsatz der Blausäure hergestellt wurde.

16. Verfahren gemäß Anspruch 13 oder 14, **dadurch gekennzeichnet, dass** in Verfahrensschritt VI zunächst aus dem in Verfahrensschritt V gewonnenen Methanol zusammen mit Schwefelwasserstoff, welches optional unter Verwendung eines Teilstroms des Prozessgases II hergestellt wurde, Methylmercaptan erhalten wird, welche in einem Folgeschritt mit der in Verfahrensschritt I hergestellten Blausäure zu Methionin umgesetzt wird.

17. Verfahren gemäß mindestens einem der Ansprüche 2 bis 12, **dadurch gekennzeichnet, dass** es sich bei Verfahrensschritt V um eine Fermentation und bei der organischen Verbindung um Aceton handelt, und dass es sich bei dem Folgeprodukt in Verfahrensschritt VI um Methacrylamid, aus welchem wiederum ein Alkylmethacrylat oder Methacrylsäure hergestellt wird, handelt, wobei das Aceton mit Blausäure bei einer Temperatur zwischen 0 °C und 60 °C mit einem basischen Katalysator in Kontakt gebracht wird, und das so erzeugte Acetoncyanhydrin nach Aufreinigung mit Schwefelsäure, Methanol und Wasser in mindestens zwei Stufen zu einem Methacrylat umgesetzt wird.

18. Verfahren gemäß mindestens einem der Ansprüche 2 bis 12, **dadurch gekennzeichnet, dass** es sich bei Verfahrensschritt V um eine Fermentation und bei der organischen Verbindung um Ethanol oder Propanol handelt, und dass es sich bei dem Folgeprodukt in Verfahrensschritt VI um den Ethylmethacrylat oder um Propylmethacrylat handelt, welche durch Veresterung eines auf Blausäure aus Verfahrensschritt I basierenden Methacrylsäureamid oder Methacrylsäure oder welche durch Umesterung eines auf Blausäure aus Verfahrensschritt I basierenden MMA mit dem Ethanol bzw. Propanol aus Verfahrensschritt V erhalten wird.

19. Verfahren gemäß mindestens einem der Ansprüche 2 bis 12, **dadurch gekennzeichnet, dass** es sich bei Verfahrensschritt V um eine Fermentation und bei der organischen Verbindung um Aceton handelt, und dass es sich bei dem Folgeprodukt in Verfahrensschritt VI um Isophorondiamin handelt, wobei das Isophorondiamin durch Umsetzung von Blausäure aus Verfahrensschritt I mit Isophoron, welches zumindest anteilig aus Aceton aus Verfahrensschritt V hergestellt wurde, handelt.

20. Verfahren gemäß Anspruch 13, **dadurch gekennzeichnet, dass** Prozessstrom VI einer weiteren Auftrennung unterzogen wird, wobei nach der Kondensation und einer optionalen Aufarbeitung eine vierte Fraktion, bei der es sich um ein Emissionsgas, das einen Stickoxid Anteil von kleiner 150 Vol-ppm aufweist, handelt.

## Claims

1. Process for preparing hydrogen cyanide and organic compounds including one to three carbon atoms and oxygen, **characterized in that** the process comprises the following process steps:
I. preparing a mixture of hydrogen cyanide and hydrogen as process gas I in a reaction zone A1 of a reactor A, proceeding from ammonia and a methane-containing fraction, in particular natural gas,
II. separating process gas I into a process gas II consisting predominantly of hydrogen cyanide and a process gas III consisting predominantly of hydrogen,
III. producing a process gas IV consisting predominantly of CO₂ in a reaction zone A2 of reactor A, proceeding from a methane-containing fraction,
IV. mixing at least a substream of process gas III with at least a substream of process gas IV to obtain a process gas V, and
V. converting process gas V in a reactor B to produce a process stream VI including an organic compound.

2. Process according to Claim 1, **characterized in that**, in a process step VI, at least a portion of the hydrogen cyanide obtained in process step I and at least a portion of the organic compound obtained in process step V are used to prepare a joint conversion product.

3. Process according to Claim 1 or 2, **characterized in that** process gas IV is obtained by the reaction of a methane-containing fraction, in particular natural gas, with oxygen, **in that** a substream of this process gas IV, as process gas IVa, is returned directly to reaction zone A2 and optionally mixed first with oxygen and then optionally with further methane-containing fraction before this gas mixture is returned to reaction zone A2 as process gas IVb.

4. Process according to at least one of Claims 1 to 3, **characterized in that** process step I is effected with substantial exclusion of oxygen in a reactor A in a reaction zone A1 having a platinum-containing catalyst, and **in that** the process gas I is treated before process step II with an acidic aqueous absorbent in an absorption apparatus I in such a way that remaining ammonia is separated off and obtained in the form of an aqueous ammonium sulfate-containing solution.

5. Process according to Claim 4, **characterized in that** the ammonia-depleted process gas II is treated in at least one absorption apparatus II with an aqueous, optionally carbonyl-containing absorbent so as to obtain an aqueous HCN-containing solution, and **in that** this solution is then treated in at least one distillation column I in such a way that a liquid product fraction I containing at least 99% by weight of hydrogen cyanide is obtained after condensation.

6. Process according to at least one of Claims 1 to 5, **characterized in that** process gas V is a synthesis gas having a molar ratio of CO and CO₂ to hydrogen between 1:1 and 1:10.

7. Process according to at least one of Claims 1 to 6, **characterized in that** process gas III has a hydrogen content > 90% by volume and **in that** this content is optionally increased between process step II and process step IV to > 95% by volume in one or more workup steps, and **in that** the optionally enriched process gas III is obtained at a pressure < 5 bara.

8. Process according to at least one of Claims 1 to 7, **characterized in that** the methane-containing fractions are natural gas, where a mixture of oxygen and process gas IVa, optionally with hydrogen, is fed into the methane-containing fractions in or upstream of reaction zone A2, and **in that** process step III serves to provide thermal energy for the endothermic reaction of process step I.

9. Process according to at least one of Claims 1 to 8, **characterized in that** the process gas IV after exiting from reaction zone A2 is at a temperature between 700°C and 1300°C and has an oxygen content of less than 5% by volume, an NOₓ content of less than 500 ppm by volume and a total content of CO, CO₂ and water of 50% to 99% by volume, and **in that** process gas IV is then cooled to a temperature between 150°C and 450°C to produce mid-pressure steam.

10. Process according to at least one of Claims 1 to 9, **characterized in that** process stream VI has an oxygen content of less than 0.1% by volume, preferably less than 0.01% by volume.

11. Process according to at least one of Claims 1 to 10, **characterized in that** one substream of process gas IV is guided into reaction zone A2 for process step III, and a second substream of process gas IV into reactor B for process step V.

12. Process according to at least one of Claims 1 to 11, **characterized in that** process gas IV before process step IV, or a substream of process gas IV guided into reactor B at a later stage, or process gas V before process step V, is subjected to deoxygenation, preferably adiabatically in contact with a transition metal catalyst or precious metal catalyst at a temperature between 200 and 800°C.

13. Process according to at least one of Claims 2 to 12, **characterized in that** the organic compound which is formed in process step V is methanol, where the conversion in process step V is effected by conversion in the gas phase over a heterogeneous catalyst at pressures of 20 to 120 bar and a temperature between 130°C and 350°C, and wherein, after process step V, process stream VI is separated by condensation into a water phase, a phase consisting predominantly of methanol, and an uncondensed process gas VII.

14. Process according to at least Claim 13, **characterized in that** process gas VII is mixed with process gas V, and **in that** reactor B comprises a plurality of series-connected subreactors, preferably shell-and-tube reactors and/or tray reactors, with achievement of a partial conversion between 20% and 90% in the respective subreactors, and where the respective gas mixture is cooled before entering the respective next subreactor.

15. Process according to Claim 13 or 14, **characterized in that** the hydrogen cyanide prepared in process step I and the methanol obtained in process step V are used to prepare methyl methacrylate in process step VI, where the methanol has been added to a mixture comprising sulfuric acid, ammonium hydrogensulfate, methacrylamide and water at a temperature between 80°C and 130°C and the methacrylamide has been prepared beforehand using the hydrogen cyanide.

16. Process according to Claim 13 or 14, **characterized in that** the methanol obtained in process step V together with hydrogen sulfide that has optionally been prepared using a substream of process gas II are first used to obtain methyl mercaptan in process step VI, and the methyl mercaptan is then reacted in a subsequent step with the hydrogen cyanide produced in process step I to give methionine.

17. Process according to at least one of Claims 2 to 12, **characterized in that** process step V is a fermentation and the organic compound is acetone, and **in that** the conversion product in process step VI is methacrylamide, from which an alkyl methacrylate or methacrylic acid is prepared in turn, where the acetone together with hydrogen cyanide is contacted with a basic catalyst at a temperature between 0°C and 60°C, and the acetone cyanohydrin thus obtained, after purification, is reacted with sulfuric acid, methanol and water in at least two stages to give a methacrylate.

18. Process according to at least one of Claims 2 to 12, **characterized in that** process step V is a fermentation and the organic compound is ethanol or propanol, and **in that** the conversion product in process step VI is ethyl methacrylate or propyl methacrylate which is obtained by esterification of a methacrylamide or methacrylic acid based on hydrogen cyanide from process step I or which is obtained by transesterification of an MMA based on hydrogen cyanide from process step I with the ethanol or propanol from process step V.

19. Process according to at least one of Claims 2 to 12, **characterized in that** process step V is a fermentation and the organic compound is acetone, and **in that** the conversion product in process step VI is isophoronediamine, where the isophoronediamine has been prepared by reacting hydrogen cyanide from process step I with isophorone that has been at least partly prepared from acetone from process step V.

20. Process according to Claim 13, **characterized in that** process stream VI is subjected to a further separation, where after the condensation and an optional workup is a fourth fraction which is an emission gas having a nitrogen oxide content of less than 150 ppm by volume.

## Revendications

1. Procédé de préparation d'acide cyanhydrique et de composés organiques, présentant un à trois atomes de carbone et de l'oxygène, **caractérisé en ce que** le procédé comprend les étapes de procédé suivantes :
I. préparation d'un mélange d'acide cyanhydrique et d'hydrogène en tant que gaz de procédé I dans une zone de réaction A1 d'un réacteur A, partant d'ammoniaque et d'une fraction contenant du méthane, en particulier du gaz naturel,
II. séparation du gaz de procédé I en un gaz de procédé II principalement constitué d'acide cyanhydrique et en un gaz de procédé III principalement constitué d'hydrogène,
III. production d'un gaz de procédé IV principalement constitué de CO₂ dans une zone de réaction A2 du réacteur A, partant d'une fraction contenant du méthane,
IV. mélange d'au moins un flux partiel du gaz de procédé III avec au moins un flux partiel du gaz de procédé IV afin d'obtenir un gaz de procédé V et
V. transformation du gaz de procédé V dans un réacteur B afin de produire un flux de procédé VI présentant un composé organique.

2. Procédé selon la revendication 1, **caractérisé en ce que** dans une étape de procédé VI, au moins une partie de l'acide cyanhydrique obtenu dans l'étape de procédé I et au moins une partie du composé organique obtenu dans l'étape de procédé V sont utilisées pour la préparation d'un produit dérivé commun.

3. Procédé selon la revendication 1 ou 2, **caractérisé en ce que** le gaz de procédé IV est obtenu par la transformation d'une fraction contenant du méthane, en particulier du gaz naturel, avec de l'oxygène, **en ce qu'**un flux partiel de ce gaz de procédé IV est recyclé en tant que gaz de procédé IVa directement dans la zone de réaction A2 et éventuellement d'abord mélangé avec de l'oxygène et ensuite éventuellement avec de la fraction contenant du méthane supplémentaire avant le recyclage de ce mélange gazeux en tant que gaz de procédé IVb dans la zone de réaction A2.

4. Procédé selon au moins l'une des revendications 1 à 3, **caractérisé en ce que** l'étape de procédé I est effectuée, sous une large exclusion d'oxygène, dans un réacteur A dans une zone de réaction A1, présentant un contact contenant du platine et **en ce que** le gaz de procédé I est traité avant l'étape de procédé II dans un dispositif d'absorption I avec un agent d'absorption aqueux acide de telle sorte que l'ammoniac résiduel est séparé et est obtenu en tant que solution aqueuse contenant du sulfate d'ammonium.

5. Procédé selon la revendication 4, **caractérisé en ce que** le gaz de procédé II appauvri en ammoniac est traité dans au moins un dispositif d'absorption II avec un agent d'absorption aqueux, contenant éventuellement du carbonyle, de telle sorte qu'une solution aqueuse contenant du HCN est obtenue et **en ce que** cette solution est ensuite traitée dans au moins une colonne de distillation I de telle sorte qu'après la condensation, on obtient une fraction de produit I liquide contenant au moins 99% en poids d'acide cyanhydrique.

6. Procédé selon au moins l'une des revendications 1 à 5, **caractérisé en ce qu'**il s'agit, pour le gaz de procédé V, d'un gaz de synthèse présentant un rapport molaire de CO et de CO₂ à hydrogène entre 1:1 et 1:10.

7. Procédé selon moins l'une des revendications 1 à 6, **caractérisé en ce que** le gaz de procédé III présente une teneur en hydrogène > 90% en volume et **en ce que** cette teneur est augmentée entre l'étape de procédé II et l'étape de procédé IV, éventuellement en un ou plusieurs paliers de traitement, à > 95% en volume et **en ce que** le gaz de procédé III éventuellement enrichi est obtenu à une pression < 5 bars absolus.

8. Procédé selon au moins l'une des revendications 1 à 7, **caractérisé en ce qu'**il s'agit, pour les fractions contenant du méthane, de gaz naturel, les fractions contenant du méthane recevant, dans ou avant la zone de réaction A2, un mélange d'oxygène et de gaz de procédé IVa, éventuellement avec de l'hydrogène, et **en ce que** l'étape de procédé III sert à mettre à disposition de l'énergie thermique pour la réaction endothermique de l'étape de procédé I.

9. Procédé selon au moins l'une des revendications 1 à 8, **caractérisé en ce que** le gaz de procédé IV présente, après la sortie de la zone de réaction A2, une température entre 700°C et 1300°C, une teneur en oxygène inférieure à 5% en volume, une teneur en NOₓ inférieure à 500 ppm en volume et une teneur totale en CO, CO₂ et en eau de 50 à 99% en volume et **en ce que** le gaz de procédé IV est ensuite refroidi à une température entre 150°C et 450°C avec production d'une vapeur à pression moyenne.

10. Procédé selon au moins l'une des revendications 1 à 9, **caractérisé en ce que** le flux de procédé VI présente une teneur en oxygène inférieure à 0,1% en volume, de préférence inférieure à 0,01% en volume.

11. Procédé selon au moins l'une des revendications 1 à 10, **caractérisé en ce qu'**un flux partiel du gaz de procédé IV est guidé dans la zone de réaction A2 pour l'étape de procédé III et un deuxième flux partiel du gaz de procédé IV est guidé dans le réacteur B pour l'étape de procédé V.

12. Procédé selon au moins l'une des revendications 1 à 11, **caractérisé en ce qu'**avant l'étape de procédé IV, le gaz de procédé IV ou un flux partiel du gaz de procédé IV guidé ultérieurement dans le réacteur B ou, avant l'étape de procédé V, le gaz de procédé V est soumis à une désoxygénation, de préférence de manière adiabatique au contact avec un catalyseur de métal de transition ou de métal noble à une température entre 200 et 800°C.

13. Procédé selon au moins l'une des revendications 2 à 12, **caractérisé en ce qu'**il s'agit, pour le composé organique qui est formé dans l'étape de procédé V, de méthanol, la transformation dans l'étape de procédé V s'effectuant par transformation en phase gazeuse sur un contact hétérogène à des pressions de 20 à 120 bars et à une température entre 130°C et 350°C et, après l'étape de procédé V, le flux de procédé VI étant séparé par condensation en une phase aqueuse, en une phase principalement constituée de méthanol et en un gaz de procédé VII non condensé.

14. Procédé selon au moins la revendication 13, **caractérisé en ce que** le gaz de procédé VII est mélangé avec du gaz de procédé V et **en ce qu'**il s'agit, pour le réacteur B, de plusieurs réacteurs partiels traversés en série, de préférence des réacteurs à faisceau tubulaire et/ou des réacteurs à claies, une conversion partielle entre 20% et 90% étant obtenue dans les réacteurs partiels respectifs et le mélange gazeux respectif étant refroidi avant l'entrée dans le réacteur partiel consécutif respectif.

15. Procédé selon la revendication 13 ou 14, **caractérisé en ce que**, dans l'étape de procédé VI, du méthacrylate de méthyle est préparé à partir de l'acide cyanhydrique préparé dans l'étape de procédé I et du méthanol obtenu dans l'étape de procédé V, le méthanol étant ajouté à un mélange contenant de l'acide sulfurique, de l'hydrogénosulfate d'ammonium, du méthacrylamide et de l'eau, à une température entre 80°C et 130°C et le méthacrylamide ayant été préparé au préalable à l'aide de l'acide cyanhydrique.

16. Procédé selon la revendication 13 ou 14, **caractérisé en ce que**, dans l'étape de procédé VI, on obtient d'abord, à partir du méthanol obtenu dans l'étape de procédé V, conjointement avec du sulfure d'hydrogène, qui a éventuellement été préparé à l'aide d'un flux partiel du gaz de procédé II, du méthylmercaptan, qui est transformé, dans une étape consécutive avec l'acide cyanhydrique préparé dans l'étape de procédé I, en méthionine.

17. Procédé selon au moins l'une des revendications 2 à 12, **caractérisé en ce qu'**il s'agit, pour l'étape de procédé V, d'une fermentation et, pour le composé organique, d'acétone et **en ce qu'**il s'agit, pour le produit dérivé dans l'étape de procédé VI, de méthacrylamide, à partir duquel on prépare à son tour un méthacrylate d'alkyle ou de l'acide méthacrylique, l'acétone étant mise en contact avec de l'acide cyanhydrique à une température entre 0°C et 60°C avec un catalyseur basique et l'acétonecyanhydrine ainsi produite étant transformée, après purification, avec de l'acide sulfurique, du méthanol et de l'eau en au moins deux étapes en un méthacrylate.

18. Procédé selon au moins l'une des revendications 2 à 12, **caractérisé en ce qu'**il s'agit, pour l'étape de procédé V, d'une fermentation et, pour le composé organique, d'éthanol ou de propanol et **en ce qu'**il s'agit, pour le produit dérivé dans l'étape de procédé VI, de méthacrylate d'éthyle ou de méthacrylate de propyle qui est obtenu par estérification d'un amide d'acide méthacrylique ou d'acide méthacrylique basé sur l'acide cyanhydrique de l'étape de procédé I ou qui est obtenu par transestérification d'un MMA basé sur l'acide cyanhydrique de l'étape de procédé I avec l'éthanol ou, selon le cas, le propanol de l'étape de procédé **V.**

19. Procédé selon au moins l'une des revendications 2 à 12, **caractérisé en ce qu'**il s'agit, pour l'étape de procédé V, d'une fermentation et, pour le composé organique, d'acétone et **en ce qu'**il s'agit, pour le produit dérivé dans l'étape de procédé VI, d'isophoronediamine, l'isophoronediamine ayant été préparée par transformation d'acide cyanhydrique de l'étape de procédé I avec de l'isophorone qui a été préparée au moins en partie à partir d'acétone de l'étape de procédé **V.**

20. Procédé selon la revendication 13, **caractérisé en ce que** le flux de procédé VI est soumis à une autre séparation, après la condensation et un traitement éventuel, étant une quatrième fraction, pour laquelle il s'agit d'un gaz d'émission, qui présente une proportion d'oxyde d'azote inférieure à 150 ppm en volume.
